(19) Europäisches Patentamt / European Patent Office / Office européen des brevets

(11) **EP 3 363 416 B1**

(12) **EUROPÄISCHE PATENTSCHRIFT**

(45) Veröffentlichungstag und Bekanntmachung des Hinweises auf die Patenterteilung:
**11.08.2021 Patentblatt 2021/32**

(51) Int Cl.:
*A61F 9/01* (2006.01)     *A61F 9/008* (2006.01)
*A61F 9/009* (2006.01)

(21) Anmeldenummer: **18157424.5**

(22) Anmeldetag: **21.01.2010**

(54) **VORRICHTUNG UND VERFAHREN ZUM ERZEUGEN VON STEUERDATEN ZUR OPERATIVEN FEHLSICHTIGKEITSKORREKTUR EINES AUGES**

DEVICE AND METHOD FOR PRODUCING CONTROL DATA FOR THE SURGICAL CORRECTION OF DEFECTIVE EYE VISION

DISPOSITIF ET PROCÉDÉ DE PRODUCTION DE DONNÉES DE COMMANDE POUR LA CORRECTION OPÉRATOIRE D'UN DÉFAUT DE VISION D'UN OEIL

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **21.01.2009 DE 102009005482**

(43) Veröffentlichungstag der Anmeldung:
**22.08.2018 Patentblatt 2018/34**

(62) Dokumentnummer(n) der früheren Anmeldung(en) nach Art. 76 EPÜ:
**10716495.6 / 2 389 148**

(73) Patentinhaber: **Carl Zeiss Meditec AG**
**07745 Jena (DE)**

(72) Erfinder:
• **STOBRAWA, Gregor**
**07743 Jena (DE)**
• **BISCHOFF, Mark**
**07749 Jena (DE)**

(74) Vertreter: **Patentanwälte Geyer, Fehners & Partner mbB**
**Perhamerstrasse 31**
**80687 München (DE)**

(56) Entgegenhaltungen:
WO-A1-2005/011547     DE-A1-102005 013 558
DE-A1-102006 053 120     US-A1- 2004 169 820
US-A1- 2005 107 775

**Beschreibung**

Die Erfindung bezieht sich auf eine Vorrichtung zur Erzeugung von Steuerdaten zur Ansteuerung einer Lasereinrichtung zur operativen Fehlsichtigkeitskorrektur eines Auges eines Patienten, wobei die Steuerdaten zur Ansteuerung einer Lasereinrichtung ausgebildet sind, welche durch Einstrahlen von Laserstrahlung in die Hornhaut des Auges Hornhaut-Gewebe trennt, die Vorrichtung die Steuerdaten so erstellt, dass die Lasereinrichtung bei Betrieb gemäß den Steuerdaten die Laserstrahlung so abgibt, dass ein Volumen in der Hornhaut isoliert ist, dessen Entfernung aus der Hornhaut die gewünschte Fehlsichtigkeitskorrektur bewirkt, und die Vorrichtung zur Ermittlung der Steuerdaten einen Krümmungsradius berechnet, den die um das Volumen verminderte Hornhaut hat.

Die Erfindung bezieht sich weiter auf ein Verfahren zum Erzeugung von Steuerdaten zur Ansteuerung einer Lasereinrichtung zur operativen Fehlsichtigkeitskorrektur eines Auges eines Patienten, wobei die Steuerdaten zur Ansteuerung einer Lasereinrichtung ausgebildet sind, welche durch Einstrahlen von Laserstrahlung in die Hornhaut des Auges Hornhaut-Gewebe trennt, die Steuerdaten so erstellt werden, dass die Lasereinrichtung bei Betrieb gemäß den Steuerdaten die Laserstrahlung so abgibt, dass ein Volumen in der Hornhaut isoliert ist, dessen Entfernung aus der Hornhaut die gewünschte Fehlsichtigkeitskorrektur bewirkt, und zur Ermittlung der Steuerdaten ein Krümmungsradius berechnet wird, den die um das Volumen verminderte Hornhaut hat.

Der klassische Weg zur Korrektur der Fehlsichtigkeit des menschlichen Auges ist die Brille. Mittlerweile wird jedoch auch vermehrt refraktive Chirurgie eingesetzt, die durch Veränderung der Augenhornhaut eine Fehlsichtigkeitskorrektur bewirkt. Ziel der Operationsmethoden ist es dabei, die Hornhaut gezielt zu verändern, um so die Lichtbrechung zu beeinflussen. Hierfür sind unterschiedliche Operationsmethoden bekannt. Am verbreitesten ist gegenwärtig die sogenannte Laser-Insitu-Keratomileusis, die auch LASIK abgekürzt wird. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp., Irvine, USA, vertrieben wird. Nachdem die Lamelle gelöst und zur Seite geklappt wurde, ist bei der LASIK-Operation die Anwendung eines Excimer-Lasers vorgesehen, der das derart freigelegte Hornhautgewebe durch Ablation abträgt. Nachdem auf diese Art und Weise in der Hornhaut liegendes Volumen verdampft wurde, wird die Hornhautlamelle wieder auf den ursprünglichen Platz zurückgeklappt.

Die Anwendung eines Laserkeratoms zum Freilegen der Lamelle ist vorteilhaft, da die Infektionsgefahr dadurch verringert und die Schnittqualität vergrößert ist. Insbesondere kann die Lamelle mit sehr viel konstanterer Dicke hergestellt werden. Auch ist der Schnitt potentiell glatter, was spätere optische Störungen durch diese auch nach der Operation verbleibende Grenzfläche mindert. Zur Schnitterzeugung wird eine Serie optischer Durchbrüche an vorbestimmten Stellen so erzeugt, dass dadurch die Schnittfläche ausgebildet wird. Beim Laserkeratom bildet die Schnittfläche die vor dem Einsatz der Laserablation abzuklappende Lamelle.

Bei der herkömmlichen LASIK-Methode wird freigelegtes Hornhautgewebe verdampft, was auch als "Schleifen" der Hornhaut mittels Laserstrahlung bezeichnet wird. Die Volumenentfernung, die für eine Fehlsichtigkeitskorrektur notwendig ist, wird dabei für jedes Flächenelement der freigelegten Hornhaut durch die Zahl der Laserpulse und deren Energie eingestellt. Man sieht deshalb in der LASIK-Methode für den Ablationslaser ein sogenanntes shot file vor, das für verschiedene Punkte auf der Augenhornhaut festlegt, wie oft der Laserstrahl auf definierte Punkte auf der Hornhaut gerichtet werden soll und mit welcher Energie. Die Volumenentfernung wurde dabei heuristisch ermittelt, nicht zuletzt da sie sehr von der Ablationswirkung des Laserstrahls, mithin von der Wellenlänge, Fluence etc. der eingesetzten Strahlung abhängt. Auch spielt der Zustand der Augenhornhaut eine Rolle; hier ist insbesondere der Feuchtigkeitsgehalt der Augenhornhaut zu nennen. Die WO 96/11655 schildert eine Vorrichtung und ein Verfahren für die LASIK-Methode. Dabei wird insbesondere eine Formel angegeben, die aus dem vor-operativen Hornhautkrümmungsradius und der gewünschten Dioptrienkorrektur den zu erreichenden Hornhautkrümmungsradius berechnet. Eine ähnliche Berechnung ist in der EP 1153584 A1 beschrieben - ebenfalls für die Hornhautablation mittels LASIK.

Die US 5993438 schlägt vor, ein Volumen aus der Hornhaut durch Verdampfen und Absorption in der Hornhaut zu entfernen. Die DE 102005013558 A1 widmet sich ebenfalls diesem Ansatz, bei dem zuerst eine Lamelle an der Hornhautvorderseite gelöst und zur Seite geklappt wird. Anschließend wird Hornhautgewebe vom derart freigelegten Inneren der Hornhaut verdampft. Danach wird die Lamelle wieder zurückgeklappt. Die DE 102005013558 A1 schlägt Berechnungen auf Basis von Zernike-Polynomen vor, mit dem Ziel, die Tiefenschärfe zu erhöhen.

Die WO 2005/092172 offenbart, wie Brechkraftmessungen, die in einer Ebene ermittelt wurden, in eine andere Ebene transformiert werden können. Die Schrift erwähnt, dass dieses Vorgehen für verschiedene Augenbehandlungen verwendet werden kann, insbesondere für die lasergestützte Ablation.

Ein weiteres laserbasiertes, augenchirurgisches Verfahren liegt darin, das zu entfernende Hornhautvolumen nicht zu verdampfen, sondern durch einen Laserschnitt zu isolieren. Das Volumen wird also nicht mehr ablatiert, sondern in der Hornhaut durch eine dreidimensionale Schnittfläche isoliert und somit entnehmbar gemacht. Für solche Verfahren sind Erfahrungswerte, die zum Schleifen der Hornhaut mittels Ablationslaserstrahlung entwickelt wurden, nicht brauchbar. Stattdessen werden Steuerdaten für den Betrieb des Lasers zur Isolation des zu entfernenden Hornhautvolumens benötigt. In der US 6110166 und der US 7131968 B2 ist ein solches augenchirurgisches Verfahren beschrieben. Dabei sind in US 6110166 verschiedene Volumenformen gezeigt, und es ist erwähnt, dass der Fachmann das passende Volumen auswählen kann.

Die DE 102006053118 A1 schildert die Erzeugung von Steuerdaten für die volumenisolierende Fehlsichtigkeitskorrektur.

Aus der DE 102006053120 A1 und aus der DE 102006053119 A1 der Carl Zeiss Meditec AG ist es bekannt, bei der Erzeugung solcher Steuerdaten von Fehlsichtigkeitsdaten auszugehen, welche die Brechkraft einer für die Fehlsichtigkeitskorrektur tauglichen Brille angeben. Auch ist es aus der DE 102006053120 A1 bekannt, dass eine Astigmatismuskorrektur oder Korrekturen höherer Aberrationsordnungen erfolgen können. Der aus der DE 102006053120 A1 bekannte Ansatz erreicht durch die Verwendung von Fehlsichtigkeitsdaten, die für eine herkömmliche Brillenkorrektur gedacht sind, eine erhebliche Vereinfachung bei der präoperativen Augenvermessung, da die Erzeugung von Brillenkorrekturdaten in der Ophthalmologie tägliche Praxis ist. Diese Vereinfachung bedingt allerdings auch eine gewisse Beschränkung der möglichen Korrekturergebnisse, weil zwangsläufig nur Korrekturen erreicht werden können, die auch mit einer normalen Brille möglich wären. Hierbei ist auch zu berücksichtigen, dass Korrekturen, wie sie z.B. mit Gleitsichtbrillen möglich sind, für den Ansatz gemäß DE 102006053120 A1 ausscheiden, da solche Korrekturen immer davon ausgehen, dass die Sehachse je nach Blickrichtung an unterschiedlichen Stellen durch das Brillenglas fällt, was es möglich macht, für unterschiedliche Blickrichtungen (z.B. beim mehr nach unten gerichteten Lesen oder dem mehr in die Entfernung gerichteten Weitsehen) unterschiedliche optische Eigenschaften der Brille zur Wirkung bringen zu können. Dies ist bei der refraktiven Chirurgie an der Hornhaut nicht anwendbar, da durch die Augenbewegung sich die Augenhornhaut bei der Änderung der Blickrichtung selbstverständlich mitbewegt. Es gibt also, anders als bei einem Brillenglas, keine Veränderung des Durchtrittspunkts der optischen Achse durch die Hornhaut, wenn der Augapfel rotiert. Der aus der DE 102006053120 A1 bekannte Ansatz kann also folglich nur vergleichsweise einfache Brillen-Fehlsichtigkeitskorrekturdaten als Eingangsgröße bei der Steuerdaten verwenden - mit der Konsequenz entsprechend begrenzter Korrekturmöglichkeiten.

Aus der DE 10334110 A1 der Carl Zeiss Meditec AG ist es bekannt, eine Schnittfläche, die das zur Fehlsichtigkeitskorrektur zu separierende Volumen zumindest teilweise umgrenzt, dadurch zu erzeugen, dass der Fokus der Laserstrahlung entlang Höhenlinien folgenden Kreisbahnen oder entlang einer Spirale zu verstellen, die an solchen Höhenlinien orientiert ist. Dabei werden die Ebenen, in denen die Höhenlinien definiert werden, bzw. auf Basis deren die Spirale definiert wird, senkrecht zur Haupteinfallsrichtung der Bearbeitungs-Laserstrahlung orientiert. Damit erreicht man, dass die Verstellung des Fokus längs der optischen Achse, die üblicherweise durch ein verstellbares Zoom-Objektiv o. ä. vorgenommen wird, sich auf die Geschwindigkeit der Abarbeitung der Bahn möglichst gering auswirkt. Da diese Fokusverstellung in der Regel sehr viel langsamer ist, als die Ablenkung quer zur Haupteinfallsrichtung der Bearbeitungs-Laserstrahlung, erhält man insgesamt dadurch eine schnelle Erzeugung der Schnittfläche.

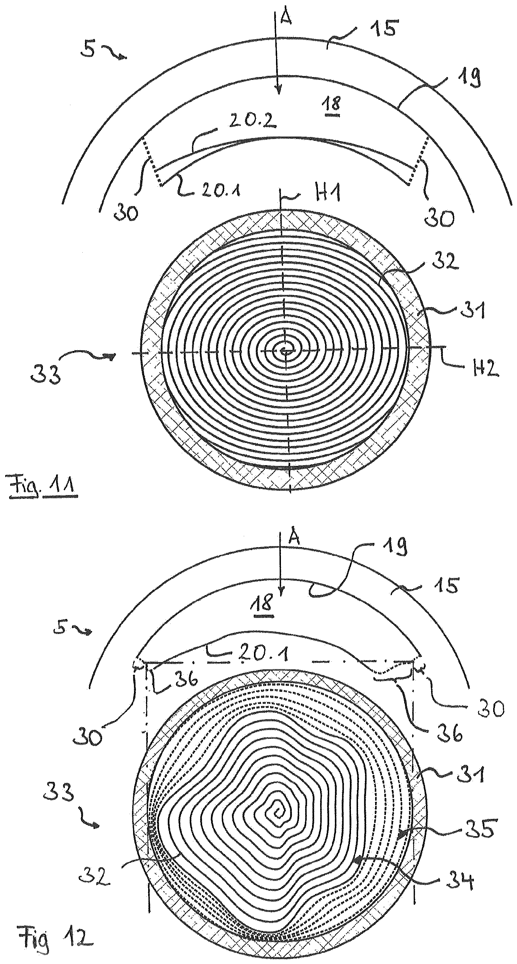

Diese Druckschrift schildert, dass bei Fehlsichtigkeitskorrekturen, die über eine sphärische Korrektur hinausgehen, beispielsweise auch einen Astigmatismus korrigieren, konsequenterweise nicht-sphärische Schnittflächen benötigt werden, beispielsweise Schnittflächen in Form eines Ellipsoides. In diesem Zusammenhang schildert es die DE 10334110 A1, dass einer solchen Schnittfläche in Sichtweise längs der Haupteinfallsrichtung der Strahlung ein kreisförmiger Umriss verliehen werden kann, wenn die Bearbeitungslaserstrahlung in Abschnitten, die über einen solchen kreisförmigen Umriss hinausgehen, deaktiviert wird. Figur 11 zeigt die dabei vorliegenden Verhältnisse. Dabei ist eine Schnittdarstellung durch eine Hornhaut 5 gezeigt, in der ein Volumen 18 isoliert und zur Entnahme vorbereitet wird. Das Volumen 18 wird dabei durch eine im Wesentlichen parallel zur Hornhautvorderfläche erzeugte anteriore Schnittfläche (Flapfläche 19) und eine posteriore Schnittfläche (Lentikelfläche 20) definiert. Im unteren Teil der Figur 11 ist eine Draufsicht 33 auf die Lentikelfläche 20 dargestellt. Sie legt die Krümmung fest, welche die Hornhautvorderseite 15 nach Entnahme des Volumens 18 hat. Figur 11 zeigt einen Fall, bei dem eine astigmatische Korrektur vorgenommen werden soll, weshalb die Lentikelfläche 20 ein Ellipsoid ist. Im oberen Teil der Figur 11 sind deshalb zwei Schnittlinien 20.1 und 20.2 für die Schnittfläche 20 dargestellt, die den Hauptachsen H1 und H2 der Ellipsoidfläche entsprechen. In der Draufsicht 33 hat das Volumen 18 einen kreisförmigen Umriss. Weiter wird die ellipsoidförmige Lentikelfläche 20 durch eine spiralförmige Bahn 32 erzeugt, entlang der die Lage des Fokus der Bearbeitungslaserstrahlung verstellt wird, auf der also die Zentren der Laserstrahlpulse liegen, welche den Bearbeitungseffekt in der Hornhaut 5 bewirken. Um einen kreisförmigen Umriss der Lentikelfläche 20 zu erreichen, wird die Bearbeitungslaserstrahlung in Bereichen der Spirale 32, die außerhalb des kreisförmigen Umrisses liegen, dunkelgetastet, d. h. so modifiziert, dass dort keine Bearbeitungswirkung eintritt. Dann kann durch eine einfache kreiskegelmantelförmige Lentikelrandfläche 30 die Verbindung zwischen der Lentikelfläche 20 und der Flapfläche 19 hergestellt werden. In der Draufsicht 33 auf die Lentikelfläche 20 ist dies durch eine kreuzschraffierte Lentikelrandzone 31 veranschaulicht, die so tief in die Augenhornhaut reicht, dass insgesamt das Volumen 18 durch die Flapfläche 19, die Lentikelfläche 20 und die Lentikelrandfläche 30 isoliert ist.

Die Druckschrift betrifft also das Konzept, eine Korrektur der optischen Abbildungsfehler des menschlichen Auges dadurch durchzuführen, dass mittels Laserstrahlung in der Augenhornhaut eine Separierung eines Gewebevolumens erreicht wird, welches dann aus der Hornhaut entfernt wird. Dadurch wird eine gezielte Änderung der Brechkraft der Augenhornhaut erreicht. Diese Änderung erfolgt lokal, d. h. in dem Bereich der Hornhaut, aus dem das Gewebevolumen entnommen wird. Üblicherweise orientiert man sich dabei an der Pupille des Auges.

Die Entnahme des separierten Volumens verändert die Geometrie, nämlich die Krümmung der Hornhautoberfläche. Damit eine gewünschte Fehlsichtigkeitskorrektur erreicht wird, muss deshalb das separierte und zu entnehmende Volumen hinsichtlich seiner Form spezielle Eigenschaften aufweisen.

In Anlehnung an das klassische LASIK-Verfahren, wird üblicherweise das separierte Volumen durch drei Grenzflächen umschrieben. Eine anteriore Grenzfläche wird in konstantem Abstand unter der Augenhornhaut ausgebildet. Dies ist dann besonders einfach, wenn die Hornhaut mit einem flachen Kontaktglas applaniert wird. Da diese Schnittfläche in die Richtung am vordersten liegt, wird sie als anteriore Fläche oder in Anlehnung an das bekannte LASIK-Verfahren als Flapfläche bezeichnet.

Weiter ist das Volumen durch eine tiefer liegende Schnittfläche begrenzt, die als posteriore Schnittfläche oder, da das Volumen als Lentikel aufgefasst werden kann, als Lentikelfläche bezeichnet wird. Es wird dafür gesorgt, dass das insgesamt zu entnehmende Volumen die Krümmung der Hornhautvorderfläche ändert. Eine der beiden Flächen, meist die posteriore, hat i. d. R. eine Geometrie, die ausschlaggebend für die Fehlsichtigkeitskorrektur ist.

Prinzipiell könnte man daran denken, die anteriore und die posteriore Fläche so zu gestalten, dass sie eine gemeinsame Schnittlinie haben. Dies ist zum einen bei einer Weitsichtigkeits-Korrektur nicht möglich, da dort das zu entnehmende Volumen in der Mitte, d. h. im Bereich der Sehachse, dünner sein muss, als am Rand. Zum anderen möchte man aus operativen Gründen auch bei einer Kurzsichtigkeits-Korrektur eine gewisse Mindestdicke des Volumens am Rande sicherstellen, um es einfach zu entnehmen können. Die anteriore Fläche und die posteriore Fläche werden deshalb über eine sogenannte Lentikelrandfläche verbunden.

Durch diese drei Schnittflächen ist das separierte Volumen entnehmbar gemacht, da dann das Volumen vollständig oder nahezu vollständig durch die Schnittflächen umschlossen ist. Die absolute Lage und relative Ausdehnung der Flächen in der Hornhaut legen die Zone fest, innerhalb der die optische Wirkung nach Entnahme des separierten Volumens zwischen diesen Flächen eintritt. Hier orientiert man sich, wie bereits erwähnt, an der Pupille. Dieser Ansatz führt dazu, dass die beiden Schnittflächen, nämlich die anteriore und die posteriore Schnittfläche, von denen eine oder beide optisch wirksam sein können, zu einem geschlossenen Volumen verbunden werden müssen, das eine geeignete Lage innerhalb der Hornhaut haben muss. Da auch gerätetechnische Randbedingungen bestehen, beispielsweise die möglichen Freiheitsgrade der Laserstrahlablenkungen, wie auch applikative Randbedingungen, wie beispielsweise Regressionseffekte während des Heilungsverlaufs, chirurgische Handhabbarkeit des zu entnehmenden Gewebevolumens, maximal tolerierbare Zeitdauer für die Erzeugung der Schnittflächen etc., ist das sich insgesamt ergebende Randwertproblem durchaus komplex.

Der Erfindung liegt die Aufgabe zugrunde, eine gattungsgemäße Vorrichtung bzw. ein gattungsgemäßes Verfahren dahingehend weiterzubilden, dass Steuerdaten für die operative Fehlsichtigkeitskorrektur möglichst rechensparsam erzeugt werden können und zugleich auch komplexere Korrekturen realisierbar sind.

Die Erfindung ist in den unabhängigen Ansprüchen definiert.

Es ist bei der Vorrichtung vorgesehen, dass der Krümmungsradius Rcv* ortsabhängig ist und folgender Gleichung genügt:

$$R_{CV}{}^*(r,\varphi) = 1 / ( (1/R_{CV}(r,\varphi)) + B_{COR}(r,\varphi)/ (n_c-1) ) + F,$$

wobei Rcv(r,(p) der lokale Krümmungsradius der Hornhaut vor Entfernung des Volumens , $n_c$ die Brechzahl des Materials der Hornhaut, F ein Faktor ist, und $B_{COR}(r,\varphi)$ die für die gewünschte Fehlsichtigkeitskorrektur nötige, lokale Brechkrafttänderung in einer im Scheitelpunkt der Hornhaut liegenden Ebene ist, wobei es mindestens zwei Radien r1 und r2 gibt, für die $B_{COR}(r=r1,\varphi) \neq B_{COR}(r=r2,\varphi)$ gilt.

Es ist beim Verfahren zum Erzeugen für Steuerdaten für eine Lasereinrichtung vorgesehen, dass der Krümmungsradius Rcv* ortsabhängig ist und folgender Gleichung genügt:

$$R_{CV}{}^*(r,\varphi) = 1 / ( (1/R_{CV}(r,\varphi)) + B_{COR}(r,\varphi)/ (n_c-1) ) + F,$$

wobei Rcv(r,(p) der lokale Krümmungsradius der Hornhaut vor Entfernung des Volumens, $n_c$ die Brechzahl des Materials der Hornhaut, F ein Faktor ist, und $B_{COR}(r,\varphi)$ die für die gewünschte Fehlsichtigkeitskorrektur nötige, lokale Brechkrafttänderung in einer im Scheitelpunkt der Hornhaut liegenden Ebene ist, wobei es mindestens zwei Radien r1 und r2 gibt, für die $B_{COR}(r=r1,\varphi) \neq B_{COR}(r=r2,\varphi)$ gilt.

Die Erfindung stellt also eine Steuergröße bzw. eine Bemessungsgröße bereit, auf deren Basis das zu entfernende Volumen und damit die dieses Volumen in der Hornhaut isolierende Schnittfläche möglichst exakt berechnet werden kann. Sie definiert eine Gleichung für den Krümmungsradius, den die Hornhaut nach der Entnahme des durch die Behandlungsvorrichtung bzw. das Verfahren isolierten Volumens haben soll. Mit dieser Gleichung ist das zu entnehmende Volumen, und insbesondere die korrekturwirksame Fläche, analytisch exakt berechenbar.

Die für die Berechnung des zu entnehmenden Volumens verwendete Gleichung unterscheidet sich bei genauer Betrachtung erheblich von dem Ansatz, wie er in der DE 102006053120 A1 verwendet wurde. Es wird nun eine andere Funktion verwendet, die nicht mehr die Brechkraft einer Brille berücksichtigt, die in Abstand zum Auge liegt, sondern eine Brechkraftverteilung, die in Kreiskoordinaten geschrieben mindestens radial variiert. Zudem liegt diese Brechkraftverteilung, mit der für das zu entnehmende Volumen der neue Krümmungsradius, den die Hornhaut nach der operativen Korrektur haben soll, berechnet wird, nicht mehr in einem Abstand zur Hornhaut, sondern gibt den Korrekturbedarf in einer Ebene an, die im Hornhautscheitel liegt. Die Erfindung greift den analytischen Ansatz der DE 102006053120 A1 auf und nimmt zugleich von den dort verwendeten Brillenkorrekturwerten Abkehr, indem nun eine radial variierende Brechkraftverteilung, welche den Korrekturbedarf in der im Hornhautscheitel liegenden Ebene wiedergibt, eingeführt wird.

Damit ist, ohne dass der Rechenaufwand signifikant gesteigert würde, eine viel weitgehendere Korrektur der Fehlsichtigkeit möglich. Beispielsweise kann nun in einem zentralen Bereich um die optische Achse, z.B. im Radius der photopischen Pupille, ein Korrekturwert angewendet werden, der dem bisherigen Brillenkorrekturwert entspricht, und für größere Durchmesser können andere Brechkraftwerte eingesetzt werden. Damit lässt sich eine Presbyopie des Auges derart beheben, dass im zentralen Bereich, z.B. im Radius der photopischen Pupille eine Korrektur für die Nahsicht (vergleichbar einer Lesebrille) und für größere Durchmesser eine Korrektur für die Fernsicht (vergleichbar einer Fernbrille) bewirkt wird.

Das Volumen bzw. die Geometrie der Korrekturwirksamen Fläche ist erfindungsgemäß über die Gleichung nun so bestimmt bzw. bestimmbar, dass die Hornhaut nach Entfernung des Volumens den definierten Krümmungsradius hat.

Eine besonders einfach zu berechnende und vor allem auch einfach zu realisierende (aber beileibe nicht die einzige) Definition des Volumens begrenzt das Volumen durch eine Grenzfläche, die in eine anteriore und eine posteriore Teilfläche (Flapfläche und Lentikelfläche) unterteilt ist, wobei die anteriore Teilfläche in konstantem Abstand $d_F$ zur Hornhautvorderfläche liegt. Die Begriffe "anterior" und "posterior" entsprechen der üblichen medizinischen Nomenklatur. Eine zusätzliche Randfläche kann erforderlich (bei Weitsichtigkeitskorrektur) bzw. vorteilhaft sein, um die beiden Teilflächen zu verbinden und zugleich eine Mindestranddicke zu gewährleisten.

Durch die in konstantem Abstand zur Hornhautoberfläche liegende anteriore Teilfläche (Flapfläche) ist die Ausbildung dieser Teilfläche besonders einfach. Die posteriore Teilfläche (Lentikelfläche) hat natürlich zwangsläufig dann keinen konstanten Abstand zur Hornhautvorderfläche. Die optische Korrektur erfolgt durch die Formgebung der posterioren Teilfläche (Lentikelfläche). Durch diesen Ansatz ist der Rechenaufwand erheblich vereinfacht, da eine sphärische Teilfläche (die anteriore Teilfläche) besonders einfach zu berechnen ist und der Rechenaufwand auf die Bestimmung der posterioren Teilfläche (Lentikelfläche) konzentriert ist. Bei einem derartigen Ansatz hat die posteriore Teilfläche (Lentikelfläche) einen Krümmungsverlauf, der bis auf eine additive Konstante identisch mit dem Krümmungsverlauf der Hornhautvorderfläche nach Entnahme des Volumens sein kann. In die Konstante fließt der Abstand, den die anteriore Teilfläche (Flapfläche) von der Hornhautvorderfläche hält, ein.

Die vorhandene radiale Abhängigkeit der Brechkraftverteilung bedeutet, dass, in Polarkoordinaten gesehen, es für alle Winkel mindestens zwei Radien gibt, bei denen unterschiedliche Werte der Brechkraftverteilung vorliegen.

Die verwendete Brechkraftverteilung kann als Ergebnis einer Berechnung unter Verwendung von Wellenfrontvermessung oder Topographiemessung der Hornhautvorderseite der Augenhornhaut vorliegen. Demgemäß sieht die erfindungsgemäße Gleichung, von welcher die Berechnung des Hornhautvolumens ausgeht, auch einen lokalen Krümmungsradius der Hornhaut vor. Das dabei gewählte Koordinatensystem ist vorzugsweise auf den Hornhautscheitel bezogen.

Ist die Topographie $Z_{CV}:R^2 \rightarrow R^3$ (Menge aller Punkte $Z_{CV}(r,\varphi)$ die auf der Hornhautvorderseite liegen) bekannt, so lässt sich der lokale Krümmungsradius $R_{CV}(z(r,\varphi))$ beispielsweise durch eine beste Anpassung einer Kugelfläche mit Radius R an die Fläche Zcv in einem infinitesimalen Radius um den Punkt $z_{CV}(r,\varphi)$ bestimmen. Auch kann nur die Anpassung eines Krümmungskreises in radialer Richtung verwendet werden. Dann gilt:

$$R_{CV}(r,\varphi) = \frac{\sqrt{1+\left(\dfrac{\partial}{\partial r}z_{CV}(r,\varphi)\right)^2}}{\left|\dfrac{\partial^2}{\partial r^2}z_{CV}(r,\varphi)\right|}$$

Auf diese Weise erhält man mittels der erfindungsgemäßen Gleichung die gewünschte Verteilung des Krümmungsradius der Hornhautvorderseite $R_{CV}{}^*(r,\varphi)$, welche durch die refraktive Korrektur $B_{COR}(r,\varphi)$ erreicht werden soll.

Das Dickenprofil $\Delta z(r,\varphi)$ des zu entnehmenden Volumens ist erfindungsgemäß nun so bestimmt bzw. bestimmbar, dass die Topographie $z_{CV}^*(r,\varphi)$ der Hornhaut nach der Entfernung des Volumens den lokalen Krümmungsradius $R_{CV}^*(r, \varphi)$ aufweist; dabei gilt:

$$z_{CV}^*(r, \varphi) = z_{CV}(r, \varphi) - \Delta z(r, \varphi).$$

Wird ein isoliertes Volumen aus der Hornhaut entfernt, so ist $\Delta z(r,\varphi)$ stets positiv. Dies ist aber keine notwendige Bedingung für die Korrektur. Es ist ebenfalls möglich, die refraktive Korrektur und damit verbunden die Veränderung der Krümmung der Hornhautvorderseite durch das Einbringen eines zusätzlichen Volumens in die Hornhaut zu verändern. In diesem Fall ist dann $\Delta z(r,\varphi)$ immer negativ. Auch gemischte Fälle sind möglich, bei denen $\Delta z(r,\varphi)$ sowohl positive als auch negative Anteile hat. Praktisch ist dies der Fall, wenn beispielsweise eine geringe refraktive Korrektur für die Fernsicht bei Myopie durch Extraktion von Gewebe und gleichzeitig eine Korrektur der Presbyopie durch Implantation einer kleinen Linse im zentralen Bereich der optischen Zone erfolgen soll. In diesem Fall kann die Dicke des Implantats durchaus größer als die Dicke des für die Myopiekorrektur zu entfernenden Gewebevolumens sein und damit $\Delta z(r,\varphi)$ im Zentralbereich positive und im Randbereich negative Werte haben.

Das Dickenprofil $\Delta z(r,\varphi)$ des Volumens ergibt sich aus der Differenz der Topographien. Ist die gewünschte Topographie nach der Korrektur $z_{CV}^*(r,\varphi)$ bekannt, so ist auch das Dickenprofil bestimmt.

Der Fachmann kann nun analytisch oder mittels geeigneter numerischer Methoden $z_{CV}^*(r,\varphi)$ aus $R_{CV}^*(r,\varphi)$ durch zweimalige Integration über die Fläche bestimmen. Die dabei anfallenden beiden Integrationskonstanten werden so gewählt, dass beispielsweise der gewünschte Behandlungsdurchmesser für die refraktive Korrektur entsteht und gleichzeitig das zu entnehmende Volumen minimiert wird.

Es ist deshalb vorgesehen, dass die Vorrichtung bei der Ermittlung der Steuerdaten die lokale Brechkraftänderung $B_{COR}(r,\varphi)$ so festlegt, dass ein charakteristischer Radius $r_{ch}$ besteht, für den die Radialfunktion der Brechkraftänderung $B_{COR}(r,\varphi)$ stückweise konstant ist, für den also $B_{COR}(r<r_{ch},\varphi=const) = B_a \neq B_b = B_{COR}(r>r_{ch},\varphi=const)$ gilt.

Die zur Korrektur verwendete Brechkraftverteilung kann, wie bereits erwähnt, in bestimmten Pupillenbereichen, z.B. einem Zentralbereich sowie einem Randbereich, unterschiedliche Werte aufweisen, um eine optische Korrektur zu erreichen, die auch bei stark variierenden Sehbedingungen optimale Ergebnisse erzielt bzw. individuell, z.B. im Fall der Altersweitsichtigkeit (Presbyopie), optimal angepasst ist.

Es ist deshalb vorgesehen, dass die Vorrichtung bei der Ermittlung der Steuerdaten die lokale Brechkraftänderung $B_{COR}(r,\varphi)$ so festlegt, dass ein charakteristischer Radius $r_{ch}$ besteht, für den die Radialfunktion der Brechkraftänderung $B_{COR}(r,\varphi)$ stückweise konstant ist, für den also $B_{COR}(r<r_{ch},\varphi=const) = B_a \neq B_b = B_{COR}(r>r_{ch},\varphi=const)$ gilt.

Zwischen den stückweise konstanten Werten der Brechkraftänderungen kann ein kontinuierlicher Übergang erfolgen. Für diese Ausgestaltung ist es deshalb zweckmäßig, dass die Vorrichtung bei der Ermittlung der Steuerdaten die lokale Brechkraftänderung $B_{COR}(r,\varphi)$ so festlegt, dass zwei Radien $r_a$ und $r_b$ bestehen, für die die Radialfunktion der Brechkraftänderung $B_{COR}(r,\varphi)$ stückweise konstant ist, für die also $B_{COR}(r<r_a,\varphi=const) = B_a \neq B_b = B_{COR}(r>r_b,\varphi=const)$ gilt, wobei die Radialfunktion der Brechkraftänderung $B_{COR}(r,\varphi)$ im Übergangsbereich zwischen $r_a$ und $r_b$ kontinuierlich von $B_a$ nach $B_b$ übergeht.

Die lokale Brechkraftänderung $B_{COR}(r,\varphi)$ kann als Spezialfall Symmetrien aufweisen, die es erlauben, die Koordinatenabhängigkeiten zu separieren. Das ermöglicht beispielsweise folgende Darstellungen bei der Steuerwerterzeugung:

$$B_{COR}(r,\varphi) = B_1(r) \cdot B_2(\varphi) \text{ (multiplikativer Separationsansatz)}$$

$$B_{COR}(r,\varphi) = B_1(r) + B_2(\varphi) \text{ (additiver Separationsansatz)}.$$

Ein Spezialfall der Separation ergibt sich, wenn die Brechkraftverteilung keine Winkelabhängigkeit aufweist. Da dies rechnerisch besonders einfach ist, ist es bevorzugt, dass bei der Ermittlung der Steuerdaten die lokale Brechkraftänderung winkelunabhängig festgelegt ist bzw. wird.

Hierbei ist ganz grundlegend darauf hinzuweisen, dass Brechkraft und Krümmungsradius mit einer einfachen Gleichung ineinander überführt werden können. Es gilt: $B = (n_c-1)/R$, wobei $B$ die Brechkraft und $R$ der für diese Brechkraft zugeordnete Radius ist. Es ist also im Rahmen der Erfindung jederzeit möglich, zwischen Radius-Betrachtungsweise und Brechkraft-Betrachtungsweise bzw. -Darstellung zu wechseln. Die bei der Ermittlung der Steuerdaten bei Brechkraft-Darstellungen zu verwendete Gleichung lautet:

$$B^{*}_{CV}(r,\varphi) = \cfrac{1}{\cfrac{1}{B_{CV}(r,\varphi) + B_{COR}(r,\varphi)} + \cfrac{F}{(n_C - 1)}}$$

Soweit hier von dem Radius der Hornhautvorderfläche gesprochen wird, kann völlig analog auch die Brechkraft verwendet werden, so dass alle hier im Zusammenhang mit dem Radius der Hornhautvorderfläche gegebene Erläuterungen selbstverständlich analog auch für die Brechkraft-Darstellung bzw. -Sichtweise gelten, wenn R durch B gemäß dem genannten Zusammenhang ersetzt wird.

Die erfindungsgemäßen Verfahren zum Erzeugen der Steuerdaten können ohne Rückgriff auf menschliche Mitwirkung ausgeführt werden. Insbesondere können sie von einem Computer durchgeführt werden, der unter Steuerung eines erfindungsgemäßen Programms das erfindungsgemäße Verfahren ausführt und aus entsprechenden Vorgaben die Steuerdaten für die Lasereinrichtung ermittelt. Insbesondere ist bei der Ermittlung der Steuerdaten die Mitwirkung eines Arztes in keiner Weise erforderlich, da mit der Ermittlung der Steuerdaten noch kein therapeutischer Eingriff verbunden ist. Dieser findet erst bei der Anwendung der zuvor ermittelten Steuerdaten statt.

Soweit in dieser Beschreibung Verfahren bzw. einzelne Schritte eines Verfahrens zur Ermittlung von Steuerdaten zur optischen Fehlsichtigkeitskorrektur beschrieben wird, kann das Verfahren bzw. können einzelne Schritte des Verfahrens durch eine entsprechend ausgestaltete Vorrichtung ausgeführt werden. Analoges gilt für die Erläuterung der Betriebsweise einer Vorrichtung, die Verfahrensschritte ausführt. Insoweit sind Vorrichtungs- und Verfahrensmerkmale dieser Beschreibung äquivalent. Insbesondere ist es möglich, das Verfahren mit einem Computer zu realisieren, auf dem ein entsprechendes erfindungsgemäßes Programm ausgeführt wird.

Auch können die hier beschriebenen Merkmale beliebig miteinander kombiniert werden, solange sie sich nicht technisch widersprechen.

Die Erfindung wird nachfolgend unter Bezugnahme auf die Zeichnungen beispielshalber noch näher erläutert. In den Zeichnungen zeigt:

Fig. 1      eine Schemadarstellung einer Behandlungsvorrichtung bzw. eines Behandlungsgerätes zur Fehlsichtigkeitskorrektur,

Fig. 2      eine Schemadarstellung hinsichtlich des Aufbaus des Behandlungsgerätes der Fig. 1,

Fig. 3      eine Prinzipdarstellung zur Einbringung gepulster Laserstrahlung in das Auge bei der Fehlsichtigkeitskorrektur mit dem Behandlungsgerät der Fig. 1,

Fig. 4      eine weitere Schemadarstellung des Behandlungsgerätes der Fig. 1,

Fig. 5      eine schematische Schnittdarstellung durch die Augenhornhaut mit Darstellung eines zur Fehlsichtigkeitskorrektur zu entfernenden Volumens,

Fig. 6      ein Schnitt durch die Augenhornhaut nach Entfernung des Volumens der Fig. 5,

Fig. 7      eine Schnittdarstellung ähnlich der Fig. 5,

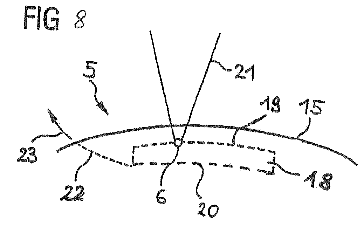

Fig. 8      eine schematische Schnittdarstellung durch die Augenhornhaut zur Veranschaulichung der Volumenentnahme,

Fig. 9      ein Diagramm mit möglichen Verläufen einer Brechkraftverteilung, welche bei der Ermittlung des zu entfernenden Volumens verwendet wird,

Fig. 10      ein Ablaufdiagramm der Ermittlung des zu entfernenden Volumens,

Fig. 11      eine Schnittdarstellung durch die Augenhornhaut zur Verdeutlichung einer anterioren sowie einer posterioren Schnittfläche in Kombination mit einer Draufsicht auf die posteriore Schnittfläche, wobei die Schnittflächengestaltungen dem Stand der Technik entsprechen,

Fig. 12      eine Schnittdarstellung durch die Augenhornhaut zur Verdeutlichung einer anterioren sowie einer posterioren Schnittfläche in Kombination mit einer Draufsicht auf die posteriore Schnittfläche, wobei eine Übergangszone zum Anpassen der Schnittfläche an einen kreisförmigen Rand vorgesehen ist,

Fig. 13      eine Darstellung ähnlich der Figur 12 für eine anders geformte Schnittfläche sowie eine andersartige Gestaltung der Übergangsfläche,

Fig. 14      eine Darstellung ähnlich der Figur 13 für eine höhere Korrekturen bewirkende Schnittfläche,

Fig. 15      eine Darstellung ähnlich der Figur 14, jedoch für eine Fehlsichtigkeitskorrektur mit applanierendem Kontaktglas,

Fig. 16      eine Darstellung ähnlich der Figur 15, jedoch ohne Übergangszone, und

Fig. 17      eine Darstellung ähnlich der Figur 16, allerdings mit einer Übergangszone, die auf einen nichtrotationssymmetrischen Rand anpasst.

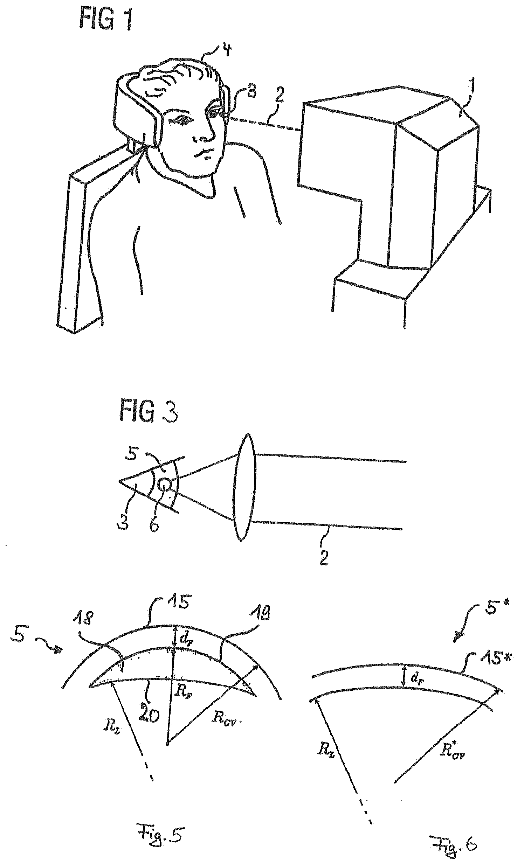

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels einer Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Aberrationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Messeinrichtungen vermessen.

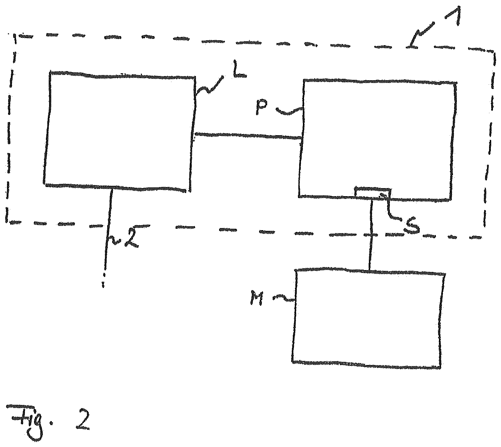

Figur 2 zeigt schematisch das Behandlungsgerät 1. Es weist in dieser Variante mindestens zwei Einrichtungen oder Module auf. Eine Lasereinrichtung L gibt den Laserstrahl 2 auf das Auge 3 ab. Der Betrieb der Lasereinrichtung L erfolgt dabei vollautomatisch, d.h. die Lasereinrichtung L startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die den Betrieb erforderlichen Steuerdaten empfängt die Lasereinrichtung L zuvor von einer Planungseinrichtung P als Steuerdatensatz über nicht näher bezeichnete Steuerleitungen. Die Übertragung findet vor dem Betrieb der Lasereinrichtung L statt. Natürlich kann Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung L gesperrt, bis an der Lasereinrichtung L ein gültiger Steuerdatensatz vorliegt. Ein gültiger Steuerdatensatz kann ein Steuerdatensatz sein, der prinzipiell zur Verwendung mit der Lasereinrichtung L der Behandlungsvorrichtung 1 geeignet ist. Zusätzlich kann die Gültigkeit aber auch daran geknüpft werden, dass weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung L ist.

Die Planungseinheit P erzeugt den Steuerdatensatz, der der Lasereinheit L zur Ausführung der Operation zur Verfügung gestellt wird, aus Messdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Messeinrichtung M, die das Auge des Patienten 4 zuvor vermessen hat. Natürlich kann die Messeinrichtung M auf beliebige Art und Weise die entsprechenden Mess- und Fehlsichtigkeitsdaten an die Planungseinrichtung P übermitteln.

Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen. Gleiches gilt natürlich hinsichtlich der Datenübertragung zwischen Planungseinrichtung P und Lasereinrichtung L.

Eine direkte Funk- oder Draht-Verbindung der Messeinrichtung M mit der Behandlungseinrichtung 1 hinsichtlich der Datenübertragung, die in einer Variante verwendet werden kann, hat den Vorteil, dass die Verwendung falscher Mess- und Fehlsichtigkeitsdaten mit größtmöglicher Sicherheit ausgeschlossen ist. Dies gilt insbesondere dann, wenn die Überführung des Patienten von der Messeinrichtung M bzw. den Messeinrichtungen zur Lasereinrichtung L mittels einer (in der Figur nicht dargestellten) Lagerungseinrichtung erfolgt, die mit der Messeinrichtung M bzw. der Lasereinrichtung L so zusammenwirkt, dass die jeweiligen Einrichtungen erkennen, ob der Patient 4 in der jeweiligen Position zum Vermessen bzw. Einbringen der Laserstrahlung 2 ist. Mit einem Verbringen des Patienten 4 von der Messeinrichtung M zur Lasereinrichtung L kann dabei zugleich auch die Übertragung der Mess- und Fehlsichtigkeitsdaten an die Behandlungsvorrichtung 1 erfolgen.

Es ist vorzugsweise durch geeignete Mittel sichergestellt, dass die Planungseinrichtung P immer den zum Patienten 4 gehörenden Steuerdatensatz erzeugt, und eine irrtümliche Verwendung eines falschen Steuerdatensatzes für einen Patienten 4 ist so gut wie ausgeschlossen.

Die Wirkungsweise des Laserstrahls 2 ist in Figur 3 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5 ein Fokus, der einen Spot 6 überdeckt und in dem die Laserstrahlungsenergiedichte so hoch ist, dass in Kombination mit der Pulslänge ein nicht-linearer Effekt im Auge auftritt. Beispielsweise kann jeder Puls der gepulsten Laserstrahlung 2 am jeweiligen Spot 6 einen optischen Durchbruch in der Augenhornhaut 5 erzeugen, welcher wiederum eine in Figur 3 schematisch angedeutete Plasmablase initiiert. Dadurch wird dieser Laserpuls in der Hornhaut 5 Gewebe getrennt. Bei Entstehung einer Plasmablase umfasst die Gewebeschichttrennung ein größeres Gebiet, als den Spot 6, welchen der Fokus der Laserstrahlung 2 überdeckt, obwohl die Bedingungen zur Erzeugung des Durchbruches nur im Fokus erreicht werden. Damit von jedem Laserpuls ein optischer Durchbruch von jedem Laserpuls erzeugt wird, muss die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, dass mehrere Laserstrahlungspulse im einen Bereich abgegeben werden, wobei sich für mehrere Laserstrahlungspulse die Spots 6 überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für die nachfolgende Beschreibung nicht weiter relevant, auch wenn in dieser Beschreibung gepulste Behandlungs-Laserstrahlung 2 geschildert wird. Beispielsweise kann ein Behandlungsgerät 1 verwendet werden, wie sie in der WO 2004/032810 A2 beschrieben ist. Eine Vielzahl von Laserpulsfoki bildet im Gewebe eine Schnittfläche aus, deren Form vom Muster abhängt, mit dem die Laserpulsfoki im Gewebe angeordnet sind/werden. Das Muster gibt Zielpunkte für die Fokuslage vor, an denen ein oder mehrere Laserpuls(e) abgegeben wird(werden), und definiert die Form und Lage der Schnittfläche.

Um nun eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, dass damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemetschen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

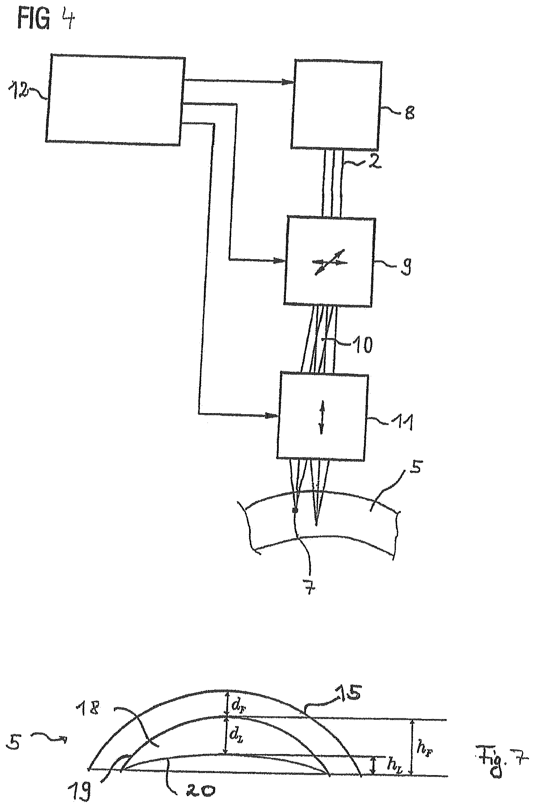

In Figur 4 sind Elemente des Behandlungsgeräts 1 nur insoweit eingetragen, als sie zum Verständnis der Fokusverstellung erforderlich sind. Die Laserstrahlung 2 wird, wie bereits erwähnt, in einem Fokus 7 in der Hornhaut 5 gebündelt, und die Lage des Fokus 7 in der Hornhaut wird verstellt, so dass zur Schnittflächenerzeugung an verschiedenen Stellen fokussiert Energie aus Laserstrahlungspulsen in das Gewebe der Hornhaut 3 eingetragen wird. Die Laserstrahlung 2 wird von einem Laser 8 als gepulste Strahlung bereitgestellt. Ein xy-Scanner 9, der in einer Variante durch zwei im Wesentlichen orthogonal ablenkende Galvanometerspiegel realisiert ist, lenkt den vom Laser 8 kommenden Laserstrahl zweidimensional ab, so dass nach dem xy-Scanner 9 ein abgelenkter Laserstrahl 10 vorliegt. Der xy-Scanner 9 bewirkt somit eine Verstellung der Lage des Fokus 7 im Wesentlichen senkrecht zur Haupteinfallsrichtung der Laserstrahlung 2 in die Hornhaut 5. Zur Verstellung der Tiefenlage ist neben dem xy-Scanner 9 ein z-Scanner 11 vorgesehen, der beispielsweise als verstellbares Teleskop ausgebildet ist. Der z-Scanner 11 sorgt dafür, dass die z-Position der Lage des Fokus 7, d.h. dessen Position auf der optischen Achse des Einfalls verändert wird. Der z-Scanner 11 kann dem xy-Scanner 9 nach- oder vorgeordnet sein. Die nachfolgend mit x, y, z bezeichneten Koordinaten beziehen sich also auf die Ablenkung der Lage des Fokus 7.

Für das Funktionsprinzip des Behandlungsgerätes 1 ist die Zuordnung der einzelnen Koordinaten zu den Raumrichtungen nicht wesentlich, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, dass eine dreidimensionale Beschreibung der Lage des Fokus 7 in der Hornhaut 5 auch durch andere Koordinatensysteme erfolgen kann, insbesondere muss es sich nicht um ein rechtwinkliges Koordinatensystem handeln. Dass der xy-Scanner 9 um zueinander rechtwinklige Achsen ablenkt, ist also nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-kartesische Koordinatensysteme zur Beschreibung bzw. Steuerung der Lage des Fokus 7 verwendet werden, wie dies nachfolgend auch noch erläutert wird. Beispiele für solche Koordinatensysteme sind Kugelkoordinaten (auch als sphärische Koordinaten bezeichnet) sowie zylindrische Koordinaten.

Zur Steuerung der Lage des Fokus 7 werden der xy-Scanner 9 sowie der z-Scanner 11, die gemeinsam ein konkretes Beispiel einer dreidimensionalen Fokusverstelleinrichtung realisieren, von einem Steuergerät 12 über nicht näher bezeichnete Leitungen angesteuert. Gleiches gilt für den Laser 8. Das Steuergerät 3 sorgt für einen geeignet synchronen Betrieb des Lasers 8 sowie der dreidimensionalen Fokusverstelleinrichtung, exemplarisch realisiert durch den xy-Scanner 9 sowie den z-Scanner 11, so dass die Lage des Fokus 7 in der Hornhaut 5 so verstellt wird, dass letztendlich ein Material bestimmten Volumens isoliert wird, wobei die spätere Volumenentfernung eine gewünschte Fehlsichtigkeitskorrektur bewirkt.

Das Steuergerät 12 arbeitet nach vorgegebenen Steuerdaten, welche die Zielpunkte für die Fokusverstellung vorgeben. Die Steuerdaten sind in der Regel in einem Steuerdatensatz zusammengefasst. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkrete Stellwerte für den Fokuslagenverstellmechanismus, z.B. für den xy-Scanner 9 und den z-Scanner 11. Zur Vorbereitung des augenchirurgischen Verfahrens, also bevor das eigentliche Operationsverfahren ausgeführt werden kann, werden die Zielpunkte und vorzugsweise auch deren Reihenfolge im Muster bestimmt. Es muss eine Vorplanung des operativen Eingriffes dahingehend erfolgen, dass die Steuerdaten für das Behandlungsgerät 1 ermittelt werden, deren Anwendung dann eine für den Patienten 4 optimale Fehlsichtigkeitskorrektur erreicht.

Zuerst gilt es das aus in der der Hornhaut 5 zu isolierende und später zu entfernende Volumen festzulegen. Wie bereits anhand Figur 2 geschildert, bedarf es dazu einer Feststellung des Korrekturbedarfs.

Hinsichtlich der in dieser Beschreibung verwendeten Nomenklatur sei angemerkt, dass durch die Anfügung eines Sterns an Größen verdeutlicht wird, dass es sich um Größen handelt, die nach einer Korrektur erhalten werden. Unter der gerechtfertigten Annahme, dass eine Dickenänderung der Hornhaut 5 im Wesentlichen den Krümmungsradius der Luft zugewandten Hornhaut-Vorderseite 15 modifiziert, nicht aber den Krümmungsradius der dem Augeninneren zuliegenden Hornhautrückseite 16, wird durch die Volumenentfernung der Krümmungsradius Rcv der Hornhautvorderseite 15 modifiziert. Die um das Volumen verminderte Hornhaut 5 mit veränderter Hornhautoberfläche 15* hat aufgrund der modifizierten Vorderseitenkrümmung eine entsprechend geänderte Abbildungswirkung, so dass ein korrigierter Fokus auf der Netzhaut 14 liegt.

Zur Bestimmung des Musters der Zielpunkte wird deshalb die zu erreichende Krümmung $R^*_{CV}$ der modifizierten Hornhautvorderfläche 15* ermittelt.

Mit dem Wert $B_{COR}$ wird nun die Krümmung der modifizierten Hornhautvorderfläche 15* wie folgt eingestellt:

$$(1) \qquad R_{CV}^*(r,\varphi) = 1 / ( (1/R_{CV}(r,\varphi)) + B_{COR}(r,\varphi)/ (n_c - 1) ) + F,$$

In Gleichung (1) bezeichnet $n_c$ die Brechkraft des Materials der Hornhaut. Der entsprechende Wert liegt üblicherweise bei 1,376; $B_{COR}$ bezeichnet eine Brechkraftänderung, die zur Fehlsichtigkeitskorrektur nötig ist. $B_{COR}$ ist radial abhängig. Unter radialer Abhängigkeit wird dabei verstanden, dass es zwei Werte r1 und r2 für den Radius r gibt, für die die Brechkraftänderung bei allen Winkeln $\varphi$ unterschiedliche Werte hat.

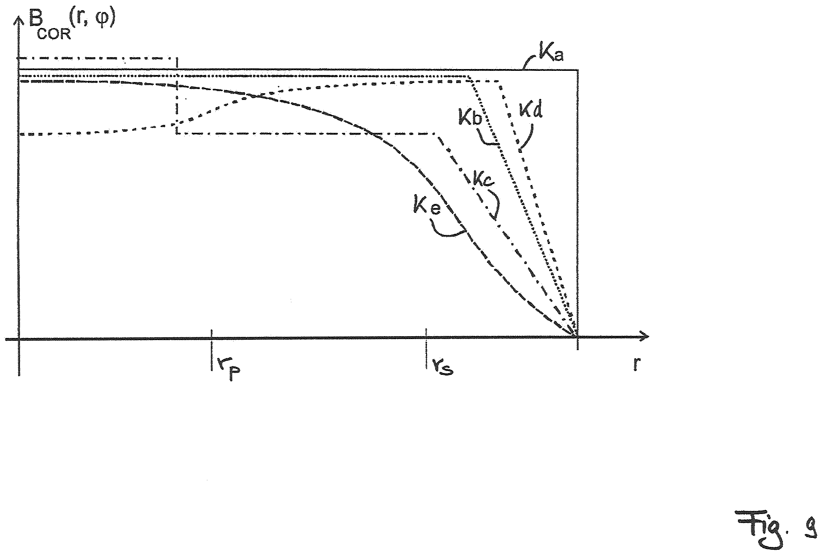

Beispiele für mögliche Verläufe der Brechkraftänderung sind in Figur 9 exemplarisch gezeigt, die die Funktion $B_{COR}$ in verschiedenen Beispielkurven Ka bis Ke als Funktion des Radius r zeigt.

Ka ist die herkömmliche Brechzahl einer Brille des Standes der Technik gemäß DE 102006053120 A1, in der Darstellung der Figur 9 jedoch bereits auf die Ebene des Hornhautscheitels bezogen. Für diesen Bezug gibt es im genannten Stand der Technik aber keinen Anlass. Er wurde hier nur zur besseren Vergleichbarkeit mit den erfindungsgemäßen beispielhaften Verläufen Kb bis Ke eingetragen. Der Verlauf Kb verläuft bis zu einem Radius, der jenseits eines Radius $r_s$ liegt, konstant und fällt dann ab. Der Radius $r_s$ ist dabei der skotopische Pupillenradius, also der Pupillenradius, der sich beim Dunkelsehen einstellt. Die Brechkraftänderung gemäß Kurve Kc ist bis zum Wert $r_s$ stückweise konstant, wobei unterhalb eines Radius $r_p$, der dem photopischen Pupillenradius entspricht, ein Sprung von einem höheren Wert auf einen niedrigeren Wert erfolgt. Eine solche Variation der Brechkraftkorrektur über dem Pupillenquerschnitt ist bei Altersweitsichtigkeit besonders vorteilhaft. Dort findet Sehen im Nahbereich üblicherweise bei guter Beleuchtung statt, z. B. beim Lesen. Aufgrund der guten Beleuchtung ist die Pupille dann in der Regel auf den photopischen Pupillenradius verengt. Der dann nötige Brechkraftkorrekturwert stellt eine optimale Anpassung an das Nahsehen ein, z. B. einen optimalen Sehabstand von etwa 25 bis 70 cm. Für den anderen Extremfall, nämlich dem Dunkelsehen, das üblicherweise mit einer Sicht in die Ferne verknüpft ist (z. B. bei nächtlichen Autofahrten) ist dagegen die Pupille maximal geöffnet. Dann wirken auch Bereiche der Pupille bei der optischen Abbildung mit, die einen anderen (z. B. niedrigeren) Wert für die Brechkraftkorrektur haben. Das menschliche Gehirn ist in der Lage, eine derart mit optischen Fehlern behaftete Abbildung (unterschiedliche Fokuslage für das Zentrum der Pupille und Randbereiche der Pupille) bei der optischen Wahrnehmung zu korrigieren. Die in den Kurven Kc oder Kd gezeigten Brechkraftkorrekturverläufe erlauben es also durch bewusstes in Kauf nehmen eines Abbildungsfehlers den Tiefenschärfebereich zu vergrößern, da der Abbildungsfehler vom Gehirn kompensiert wird. Ab dem Pupillenradius $r_s$ fällt die Brechkraftkorrektur dann weiter ab. Der nichtstufenförmige Abfall der Brechwertkorrektur auf den Wert Null ist aus anatomischer Sicht vorteilhaft. Er erlaubt am Rand des korrigierten Bereiches, d. h. am Rande des zu entfernenden Volumens, eine Anpassung des korrigierten Hornhautvorderseitenradius, welcher sich aufgrund der Korrektur einstellt, an den ursprünglichen Hornhautkrümmungsradius, d. h. den prä-operativen Radius. Bezogen auf die Darstellung der Figur 5 heißt dies, dass im Randbereich des zu entfernenden Volumens, an dem in der Darstellung der Figur 5 die Radien $R_F$ und $R_L$ zusammenlaufen, eine Angleichung dieser Radien erfolgt. Dadurch ist an der Hornhautvorderfläche nach der Korrektur der Übergang vom neuen Hornhautvorderseitenradius $R^*_{CV}$, der in dem Bereich vorliegt, in dem das Volumen 18 entnommen wurde, an den ursprünglichen Hornhautkrümmungsradius Rcv vergleichsmäßig ist. Dadurch ist die optische Korrektur insgesamt besser, was erst durch die radial variierende Brechkraftkorrektur erzielbar ist. Der Abfall der Brechkraftkorrektur auf den Wert Null erfolgt vorzugsweise in einem Bereich außerhalb des dunkel angepassten Pupillenradius, also in einem für das Sehen nicht weiter relevanten Bereichs der Augenhornhaut. Einen ähnlichen Verlauf zeigt die Kurve Kd, allerdings findet hier ein gleitender Übergang vom ersten Wert der Brechkraftänderung unterhalb $r_p$, auf den zweiten Wert, der bei $r_s$ vorliegt, statt. Zudem ist exemplarisch der erste Wert hier niedriger als der zweite Wert. Dies kann natürlich auch für die Kurve Kc so verwendet werden, je nach gewünschtem Korrekturbedarf. Einen gleitenden Verlauf, der kontinuierlich abnimmt, zeigt Kurve Ke.

Die anhand Figur 9 geschilderten örtlich abhängigen Brechkraftänderungen mit radialer Abhängigkeit sind Beispiele für eine Brechkraftänderung, die bei der Bestimmung des in der Operation zu entfernenden Volumens verwendet wird.

Der Faktor F drückt die optische Wirkung der Dickenänderung aus, welche die Hornhaut durch den operativen Eingriff erfährt und kann in erster Näherung als konstanter Faktor angesehen werden, der z. B. experimentell zuvor bestimmt werden kann. Für eine hochgenaue Korrektur kann der Faktor gemäß folgender Gleichung errechnet werden:

$$(2) \qquad F = (1 - 1/n_c) \bullet \Delta z(r=0, \varphi)$$

$\Delta z(r = 0, \varphi)$ ist dabei die zentrale Dicke des zu entfernenden Volumens.

Für eine genaue Bestimmung erfolgt eine Berechnung von $Rcv^*$ iterativ, indem bei der i-ten Berechnung aus der Differenz $1/Rcv^*(r=0,\varphi)$ - $1/Rcv(r=0,\varphi)$ auf die Größe $\Delta z(r=0,\varphi)$ geschlossen wird und das entsprechende daraus erhaltene Ergebnis für die Dickenänderung bei der (i+1)-ten Berechnung von $R^*_{CV}$ angewendet wird. Dies kann man so lange durchführen, bis ein Abbruchkriterium erfüllt wird, beispielsweise wenn die Differenz des Ergebnisses für die Dickenänderung bei zwei aufeinanderfolgenden Iterationsschritten unter einer entsprechend festgelegten Grenze liegt. Diese Grenze kann beispielsweise über eine konstante Differenz festgelegt werden, die einer für die Behandlung angemessene Genauigkeit der Refraktionskorrektur entspricht.

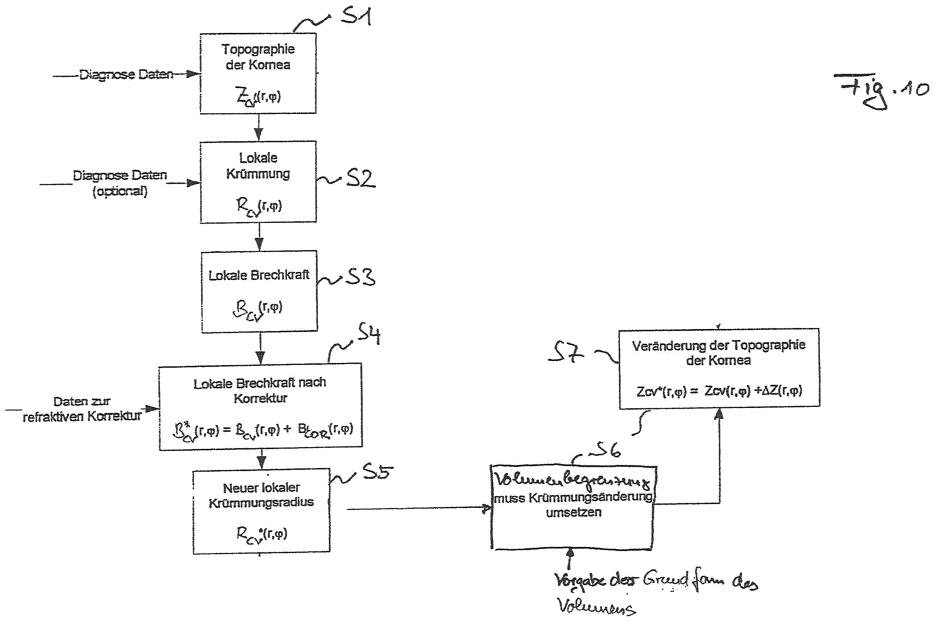

Allgemein kann dabei das in Figur 10 dargestellte Verfahren ausgeführt werden. In einem Schritt S1 wird aus Diagnosedaten die Topographie der Kornea berechnet, wie bereits eingangs im allgemeinen Teil der Beschreibung erwähnt wurde. Aus dieser Topographie wird der radiale Krümmungsverlauf der Hornhautvorderseite 15 ermittelt. Diese Ermittlung kann anstatt der Topographiedaten aus Schritt S1 auch direkt aus Diagnosedaten vorgenommen werden, so dass Schritt S2 entweder dem Schritt S1 nachgeordnet ist, oder direkt Diagnosedaten verwertet, wie Figur 10 durch die Anfügung "(optional)" verdeutlicht. Schritt S1 ist also optional.

In einem Schritt S3 wird dann die lokale Brechkraft der Kornea ermittelt.

Aus Daten der gewünschten refraktiven Korrektur wird in einem Schritt S4 die erforderliche lokale Brechkraftänderung $B_{COR}$ und mit dieser aus der lokalen Brechkraft die nach der Korrektur erwünschte lokale Brechkraft bestimmt.

Aus dieser ergibt sich in Schnitt S5 der neue lokale Krümmungsradius $R^*_{CV}(r, \varphi)$. Anstelle der Berechnung der lokalen Brechkraft Bcv in Schritt S3 kann auch direkt mit der lokalen Krümmung Rcv aus Schritt S2 gerechnet werden, wenn die obige Gleichung (1) verwendet wird. Hierbei ist ganz grundlegend darauf hinzuweisen, dass Brechkraft und Krümmungsradius mit einer einfachen Gleichung ineinander überführt werden können. Es gilt: $B = (n_c-1)/R$, wobei B die Brechkraft und R der für diese Brechkraft zugeordnete Radius ist. Es ist also im Rahmen der Erfindung jederzeit möglich, zwischen Radius-Betrachtungsweise und Brechkraft-Betrachtungsweise bzw. -Darstellung zu wechseln. Die bei der Ermittlung der Steuerdaten bei Brechkraft-Darstellungen zu verwendende Gleichung lautet:

$$B^*{}_{CV}(r,\varphi) = \cfrac{1}{\cfrac{1}{B_{CV}(r,\varphi) + B_{COR}(r,\varphi)} + \cfrac{F}{(n_C - 1)}}$$

Soweit hier von dem Radius der Hornhautvorderfläche gesprochen wird, kann völlig analog auch die Brechkraft verwendet werden, so dass alle hier im Zusammenhang mit dem Radius der Hornhautvorderfläche gegebene Erläuterungen selbstverständlich analog auch für die Brechkraft-Darstellung bzw. -Sichtweise gelten, wenn R durch B gemäß dem genannten Zusammenhang ersetzt wird.

Für das Volumen, dessen Entfernung die obige Krümmungsänderung der Hornhautvorderfläche 15 bewirkt, wird nun in einem Schritt S6 die das Volumen isolierende Grenzfläche festgelegt. Dabei ist zu berücksichtigen, welche Grundform das Volumen haben soll.

In einer ersten Variante wird mittels dem Fachmann bekannter numerischer Methoden eine Freifläche definiert, die das Volumen umschreibt, dessen Entfernung die Krümmungsänderung bewirkt. Dazu wird entlang der z-Achse die Volumendicke ermittelt, die zur gewünschten Krümmungsmodifikation nötig ist. Daraus ergibt sich das Volumen als Funktion von r, $\varphi$ (in Zylinderkoordinaten) und daraus wiederum dessen Grenzfläche.

Eine analytische Rechnung liefert hingegen die folgende, bereits in der DE 102006053120 A1 angesprochene Variante, bei der die Grenzfläche des Volumens im Wesentlichen durch zwei Teilflächen aufgebaut wird, eine zur Hornhautoberfläche 15 hin liegende anteriore Teilfläche und eine gegenüberliegende posteriore Teilfläche. Die entsprechenden Verhältnisse zeigt Figur 5. Das Volumen 18 ist zur Hornhautvorderfläche 15 hin durch eine anteriore Schnittfläche 19 begrenzt, die in konstantem Abstand $d_F$ unter der Hornhautvorderfläche 15 liegt. Diese anteriore Schnittfläche 19 wird in Analogie zur Laserkeratomen auch als Flapfläche 19 bezeichnet, da sie dort dazu dient, in Kombination mit einem Öffnungsschnitt zum Rand hin die Augenhornhaut 5 eine Lamelle in Form eines "Flap" von der darunterliegenden Hornhaut 5 abheben zu können. Diese Art der Entnahme des zuvor isolierten Volumens 18 ist natürlich auch hier möglich.

Die anteriore Schnittfläche 19 ist bevorzugt sphärisch, da dann für sie ein Krümmungsradius angegeben werden kann, der um die Lamellendicke $d_F$ geringer ist als der Krümmungsradius Rcv.

Posterior ist das Volumen 18, das aus der Hornhaut 5 entfernt werden soll, durch eine posteriore Schnittfläche 20 begrenzt, die schon grundsätzlich nicht zur Hornhautvorderfläche 15 in konstantem Abstand sein kann. Die posteriore Schnittfläche 20 wird deshalb so ausgebildet sein, dass das Volumen 18 in Form eines Lentikels vorliegt, weshalb die posteriore Schnittfläche 20 auch als Lentikelfläche bezeichnet wird. In Figur 5 ist sie exemplarisch als ebenfalls sphärische Fläche mit einem Krümmungsradius $R_L$ eingezeichnet, wobei natürlich das Zentrum dieser Krümmung nicht mit dem Krümmungszentrum der in Figur 5 ebenfalls sphärischen Hornhautvorderfläche 15 zusammenfällt. Am Rand werden die beiden Flächen 19, 20 vorzugsweise durch eine Lentikelrandfläche verbunden, um das zu entnehmende Volumen vollständig zu umgrenzen und zugleich eine Mindestdicke am Rand zu gewährleisten.

Figur 6 zeigt die Verhältnisse nach Entfernung des Volumens 18. Der Radius der modifizierten Hornhautvorderfläche 15* beträgt nun Rcv* und kann beispielsweise gemäß den zuvor beschriebenen Gleichungen berechnet werden. Die Dicke $d_L = \Delta z(r=0,\varphi)$ des entnommenen Volumens 18 ist dabei maßgeblich für die Radiusänderung, wie Figur 7 verdeutlicht. In dieser Figur ist vereinfacht die Lentikelfläche sphärisch. Folglich sind als weitere Größen noch die Höhe $h_F$ der durch die Flapfläche 19 definierten Kugelkappe, die Höhe $h_L$ der durch die Lentikelfläche 20 definierten Kugelkappe sowie die Dicke $d_L$ des zu entfernenden Volumens 18 eingezeichnet.

Die Lentikelfläche 20 legt aufgrund des konstanten Abstandes zwischen Hornhautvorderfläche 15 und Flapfläche 19 den Krümmungsverlauf der Hornhautvorderfläche 15* nach Entfernung des Volumens 18 fest.

Soll der Faktor F bei der Berechnung berücksichtigt werden, wird in Schritt S7 noch die Veränderung der Topographie der Kornea berücksichtigt, d.h. die aktuelle Mittendicke berechnet. Mit dem sich daraus ergebenden Wert für den Faktor F können dann die Schritte S4 bis S6 oder S5 bis S6 nochmals oder mehrmals in Form einer Iteration durchlaufen werden.

Die in den Figuren gezeigte Ausbildung des Volumens 18 als durch eine Flapfläche 19 mit konstantem Abstand zur Hornhautvorderfläche 15 sowie eine Lentikelfläche 20 begrenzt, ist nur eine Variante zur Begrenzung des Volumens 18. Sie hat jedoch den Vorteil, dass die optische Korrektur wesentlich nur durch eine Fläche (die Lentikelfläche 20) festgelegt wird, so dass die analytische Beschreibung der anderen Teilfläche der Grenzfläche einfach ist.

Weiter sind optimale Sicherheitsmargen hinsichtlich des Abstandes des Volumens zur Hornhautvorderfläche 15 und Hornhautrückfläche 16 gegeben. Die Restdicke $d_F$ zwischen Flapfläche 19 und Hornhautvorderfläche 15 kann konstant auf einen Wert von beispielsweise 50 bis 200 $\mu$m eingestellt werden. Insbesondere kann sie so gewählt sein, dass das schmerzempfindliche Epithel in der Lamelle verbleibt, die durch die Flapfläche 19 unter der Hornhautvorderfläche 15 gebildet ist. Auch steht die Ausbildung der sphärischen Flapfläche 19 in Kontinuität mit bisherigen Keratometerschnitten, was für die Akzeptanz der Methode vorteilhaft ist.

Nach Erzeugen der Schnittflächen 19 und 20 wird dann das derart isolierte Volumen 18 aus der Hornhaut 5 entfernt. Dies ist schematisch in Figur 9 dargestellt, die zudem verdeutlicht, dass die Schnittflächen 19 und 20 durch Einwirkung des in einem Fokuskegel 21 einfallenden Behandlungslaserstrahls erzeugt werden, beispielsweise durch Aneinanderreihung von Plasmablasen, so dass in einer bevorzugten Ausführungsform die Flapfläche 19 und die Lentikelfläche 20 durch geeignete dreidimensionale Verstellung der Fokuslage der gepulsten Laserstrahlung 2 erzeugt werden.

Erzeugt man sowohl die Lentikelfläche 20 als auch die Flapfläche 19 mittels gepulster Laserstrahlung, ist es zweckmäßig, die Lentikelfläche 20 vor der Flapfläche 19 auszubilden, da das optische Ergebnis bei der Lentikelfläche 20 besser (wenn nicht überhaupt erst zu erreichen) ist, wenn oberhalb der Lentikelfläche 20 noch keine Veränderung der Hornhaut 5 eintrat.

Figur 12 zeigt eine Darstellung, die in ihrem oberen Teil von der Art der Ansicht der Figur 5 entspricht. Im unteren Teil ist eine Draufsicht 33 auf die Lentikelfläche 20 gezeigt, die in der darüber liegenden Schnittdarstellung nur durch eine Schnittlinie 20.1 veranschaulicht ist.

Zur Isolierung des Volumens 18 wird zum einen die Flapfläche 19 als auch die Lentikelfläche 20 in der Augenhornhaut 5 auf die geschilderte Art und Weise erzeugt. Dabei wird eine Korrekturfläche erzeugt, die nicht rotationssymmetrisch ist, da sie höhere Aberrationen korrigieren soll, die Krümmung der Vorderseite 15 der Hornhaut 5 nach der Entnahme des Volumens 18 also nicht nur hinsichtlich der Sphärizität geändert werden soll. Diese Korrekturfläche wird, wie die Draufsicht 33 auf die Lentikelfläche 20 zeigt, durch eine Spirale 32 erzeugt, die vom Inneren der Korrekturfläche nach außen läuft. Die Spirale definiert eine Bahnkurve für die Verstellung der Lage des Laserstrahlfokus. Das Zentrum der Spirale liegt dabei vorzugsweise (aber nicht zwingend) am höchsten Punkt der Korrekturfläche. Die Spirale basiert auf Höhenlinien, wodurch die z-Position (Position längs der Haupteinfallsrichtung A der Laserstrahlung) der Fokuslage kontinuierlich verstellt wird. Anstelle einer Gruppe von geschlossenen Scanlinien, die sich niemals schneiden, liegt eine kontinuierliche Scanlinie vor. Lokale ortsabhängige Brechtkraftkorrekturen $B(r, \varphi)$ lassen sich durch die Modulation der winkelabhängigen Radialfunktion $r(\varphi)$ durch eine derart radial "deformierte" Spirale einfach darstellen und erzeugen.

Die Randlinie der Lentikelfläche 20 soll eine Kreislinie sein, die in z-Richtung, die, wie üblich, die Haupteinfallsrichtung A der bearbeitenden Laserstrahlung ist, in einer Ebene liegt. Für den Rand $r_{MAX'}$ ($f_P$, $\varphi$) der Lentikelfläche 20 gilt also z = konst. Die Korrekturfläche, welche für die optische Korrektur erforderlich ist, ist in einem Korrekturbereich 34 definiert. Hier ist die Bahnkurve durchgezogen dargestellt. Der Rand dieses Korrekturbereichs ist natürlich nicht rotationssymmetrisch - wohl aber eben, da die Spirale auf Höhenlinien basiert. Es erfolgt deshalb in einem Übergangsbereich 35 eine Modifikation der Spirale derart, dass innerhalb einer begrenzten Anzahl von Umdrehungen der winkelabhängige Bahnabstand so moduliert wird, dass der nicht-rotationssymmetrische Rand der Korrekturfläche in einen Kreis übergeht. Die radiale Modulation wird also über eine bestimmte Anzahl von Umdrehungen auf Null reduziert. Beispielsweise kann dies dadurch erfolgen, dass die Anzahl der Umdrehungen der Spirale im Übergangsbereich so gewählt wird, dass sie dem Quotienten aus der Radiendifferenz und dem gewünschten Abstand der Spiralbahnen entspricht. Die Radiendifferenz ist dabei der Unterschied zwischen dem minimalen Radius der Korrekturfläche und dem Radius des gewünschten kreisförmigen Randes, welcher vorzugsweise dem maximalen Radius der Korrekturfläche gleicht oder nur geringfügig größer ist.

Durch diese Fortsetzung der Spirale im Übergangsbereich 34 wird die Korrekturfläche, welche die Schnittflächengeometrie im Korrekturbereich 34 ist, so fortgesetzt, dass sie in einem kreisförmigen Rand endet. Dies ist in den Verhältnissen der Schnittdarstellungen gut zu erkennen, in die zur Verdeutlichung strichpunktiert Bezugslinien eingezeichnet sind. Weiter ist die Fortsetzung der Korrekturfläche im Übergangsbereich in der Schnittdarstellung mit der gleichen gestrichelten Linie dargestellt, wie die entsprechenden Spiralenumdrehungen in der Draufsicht 33 auf de Lentikelfläche 20. Die Schnittdarstellung zeigt, dass die Enden der Lentikelfläche 20 in einer Ebene liegen. Weiter sind sie kreisförmig. Es kann deshalb mit einer einfachen, kreiskegelmantelförmigen Lentikelrandfläche 30 die Verbindung zwischen der Lentikelfläche 20 und der sphärischen Flapfläche 19 hergestellt werden.

Dabei gibt es keine Abschnitte der Lentikelrandfläche 30 oder der Flapfläche 19, die in die Hornhaut eingebracht würden und die nicht zur Verbindung mit der Lentikelfläche 20 benötigt werden.

Für das Verständnis der hier geschilderten Ausführungsformen ist es wesentlich, zwischen dem Übergangsbereich 35 und dem Lentikelrandbereich 31 (entsprechend den Schnittflächen 36 und 30) zu unterscheiden. Die Übergangszone passt die ansonsten nicht-rotationssymmetrische Korrekturfläche so an, dass die Lentikelfläche 20 insgesamt einen rotationssymmetrischen Rand hat. Dieser Rand liegt dabei nicht tiefer, d. h. posteriorer als die für die Korrekturfläche (entsprechend der Schnittlinie) aber auch nicht höher, d. h. anteriorer. Die Ebene, in der der kreisförmige Rand durch den Übergangsbereich 35 ausgebildet wird, schneidet also die Korrekturfläche oder liegt zumindest auf dem Maximum oder Minimum dieser Fläche. Die Korrekturfläche wird also durch die Übergangszone ergänzt, ist aber von der Lentikelrandfläche zu unterscheiden, die als einfache kreiszylinder- oder -kegelmantelförmige Schnittfläche die Verbindung zwischen zwei rotationssymmetrischen Rändern, nämlich dem der Lentikelfläche 20, welcher durch die Übergangszone 35 erreicht wurde, und dem der (in der geschilderten Ausführungsform schon ohnehin sphärischen) Flapfläche 19.

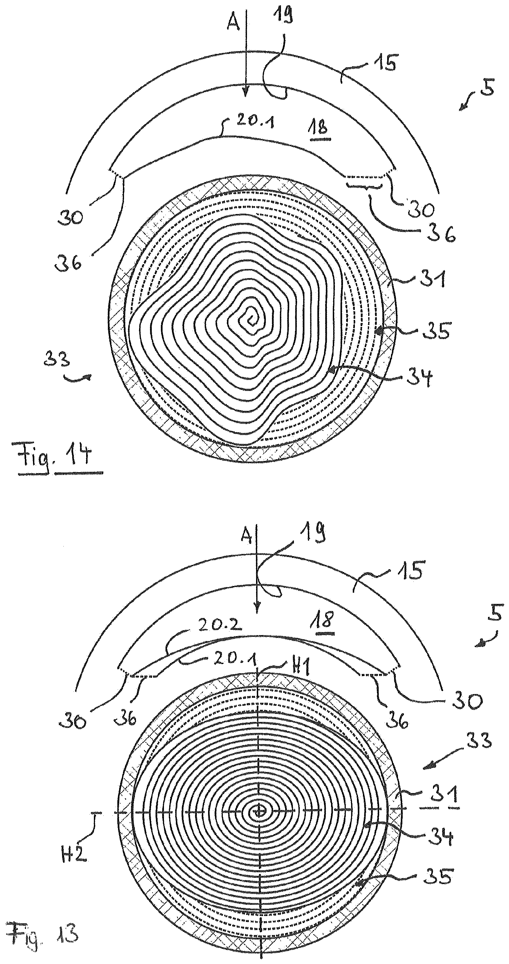

Figur 12 zeigt eine Ausführungsform, bei der die Übergangszone 35 eine kontinuierliche und glatte, z. B. differenzierbare, Anpassung zwischen der Randfläche der Korrekturfläche (Schnittfläche in der Korrekturzone 34) und dem kreisförmigen Rand vornimmt. Ein solcher glatter Verlauf ist jedoch nicht zwingend erforderlich, wie Figur 14 zeigt.

In Figur 13 ist die Korrekturzone 34 in diesem Fall durch die Korrekturfläche vorgegeben, welche beispielshalber zur Astigmatismuskorrektur als Ellipsoid ausgebildet ist. Die Schnittdarstellung der Lentikelfläche 20 zeigt deshalb zwei Schnitte 20.1 und 20.2, die den Halbachsen H1 und H2 der Ellipsoid-Fläche in der Korrekturzone 34 entsprechen. Auch wird nun eine andere Art und Weise gewählt, die Korrekturzone 34 so durch die Übergangszone 35 zu ergänzen, dass insgesamt ein rotationssymmetrischer, d. h. kreisförmiger Rand vorliegt. Wiederum wird die Lentikelfläche 20 durch eine spiralförmige Bahn, entlang der der Fokus der Laserstrahlung abgelenkt wird, erzeugt, wie dies aus der Draufsicht 33 erkennbar ist. Ist der Rand der Korrekturzone 34 erreicht, der, wie bereits im allgemeinen Teil der Beschreibung erwähnt, entweder durch den Rand der vorgegebenen Korrekturfläche oder dadurch gegeben ist, dass eine größere Korrekturfläche über den gewünschten Pupillenquerschnitt erzeugt ist, wird der spiralförmige Verlauf der Bahn auf eine kreisförmige Spirale mit konstantem z-Wert umgeschaltet.

Es liegt also in der Übergangszone 35 eine Spirale mit konstantem Bahnabstand vor, die vom kleinsten Radius der Korrekturfläche in der Korrekturzone 34 bis zum Radius des rotationssymmetrischen Randes geführt wird, der zweckmäßigerweise mit dem größten Radius des Randes der Korrekturzone 34 gleichgesetzt wird. Gegebenenfalls kann jedoch die Übergangszone noch eine gewisse Zugabe erhalten, der Radius des rotationssymmetrischen Randes also um ein Zugabemaß größer gewählt werden, als der größte Radius der Korrekturfläche in der Korrekturzone 34.

Beim Abarbeiten dieser Spirale mit konstantem Bahnabstand wird allerdings an denjenigen Bahnabschnitten eine Laserbearbeitung unterdrückt, deren Position innerhalb der Korrekturzone bzw. innerhalb des Randes der Korrekturzone 34 lägen. Im Falle einer Laserbearbeitung durch gepulste Laserstrahlung werden beispielsweise mit dem Konzept der DE 10358927 A1, auf deren Offenbarung diesbezüglich verwiesen wird, die Laserstrahlungspulse hinsichtlich ihrer Bearbeitungswirkung "unschädlich" gemacht. In der Schnittdarstellung der Figur 13 zeigt sich, dass durch die Spirale mit konstantem Bahnabstand und festem z-Wert die Übergangszone 34 in einer Fortsetzung der posterioren Schnittfläche resultiert, die als Übergangsschnittfläche 36 eingetragen ist und senkrecht zur Einfallsrichtung A der Laserstrahlung liegt. Die Ausdehnung dieser Übergangsschnittfläche 36 hängt natürlich vom Abstand des Randes der nicht-rotationssymmetrischen Korrekturfläche bzw. Korrekturzone 34 vom rotationssymmetrischen Rand ab. Dies führt dazu, dass die Übergangsschnittfläche 36 auf der rechten Seite in Figur 14 in der Schnittdarstellung sehr viel länger ist, als auf der linken Seite, wo sie nahezu punkförmig ist, da der rotationssymmetrische Rand nahezu gleich dem maximalen Radius der Korrekturzone 34 gewählt wurde.

Die Darstellung in Figur 14 entspricht im Wesentlichen der der Figur 13. Allerdings ist die Korrekturfläche bzw. die Korrekturzone 34 hier nicht ellipsoidförmig, also in der Draufsicht 33 nicht elliptisch, sondern zur Korrektur höherer Aberrationen geeignet ausgebildet. Ansonsten gilt das oben zur Figur 13 gesagte uneingeschränkt auch für die Figur 14, die zeigt, dass das Konzept der Übergangszone der Figur 13 nicht zwingend mit einer ellipsoidförmigen Korrekturfläche verbunden sein muss.

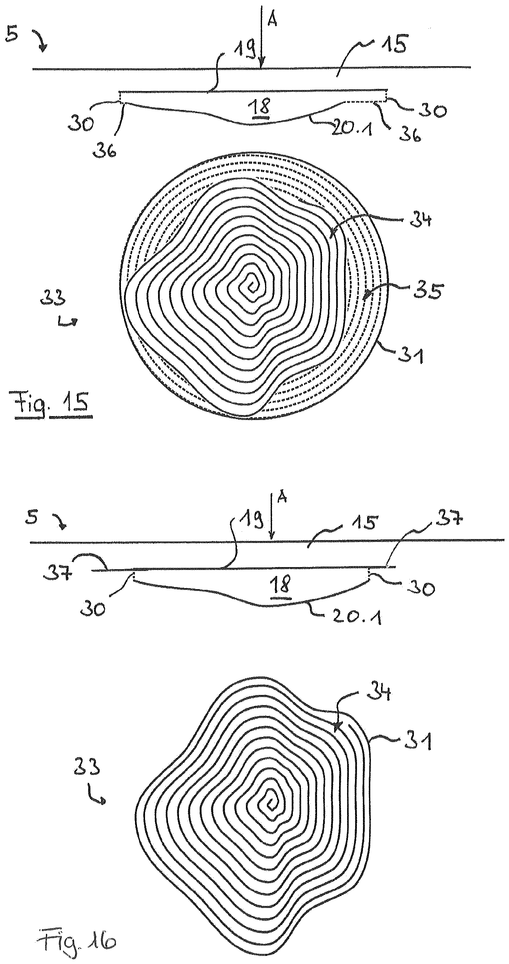

Figur 15 zeigt eine Ausführungsform, bei der die Hornhaut 5 mittels eines ebenen Kontaktglases applaniert wird. Die Flapfläche 19 ist deshalb als Ebene ausgebildet. Auch erscheint die Lentikelrandzone 31 in der Draufsicht 33 nur noch als Linie. Die Übergangszone 35 ist analog zur Ausführungsform der Figuren 13 und 14 als ebene Spirale mit konstantem Bahnradius ausgebildet. Das hinsichtlich der Figuren 13 und 14 erläuterte gilt somit in gleichem Maße.

Die Übergangszone 35 ist also eine planare Spirale mit konstantem Bahnabstand, der von der kleinen Halbachse H1 bis zur großen Halbachse H2 der elliptischen Korrekturzone 34 verläuft, um auf den kreisförmigen Rand zu gelangen.

In der Ausführungsform der Figur 15 ist die Lentikelrandfläche als Kreiszylinder ausgeführt, in dem dafür gesorgt ist, dass der Randradius der Flapfläche 19 gleich dem Randradius der Lentikelfläche 20 ist und das weiter die Ränder genau untereinander liegen. Dies ist jedoch nicht zwingend erforderlich. Man kann sowohl unterschiedliche Radien verwenden, also auch die kreisförmigen Ränder gegeneinander versetzen. Dann sind für die Lentikelfläche Kreisschrägzylinder bzw. Schrägkegelmantelflächen erforderlich.

Figur 16 zeigt eine Ausführungsform, bei der keine Übergangszone 35 vorgesehen wird. Statt dessen wird direkt vom nicht-rotationssymmetrischen Rand der Korrekturzone 34 eine entsprechend auch nicht-rotationssymmetrische Lentikelrandfläche 30 zur Flapfläche 19 ausgebildet. Bei dieser Fläche handelt es sich dann um eine Zylinderfläche, deren Erzeugende dem Rand der Korrekturzone 34 entspricht.

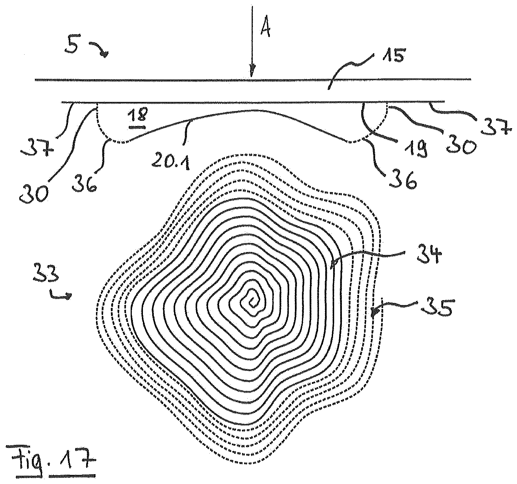

Die Ausführungsform der Figur 17 sieht eine Übergangszone 35 vor, die die Korrekturzone 34 durch Verringerung der z-Koordinate so fortsetzt, dass die Übergangszone 35 unter Beibehaltung des nicht-rotationssymmetrischen Umfanges direkt bis zur Flapfläche 19 geführt wird. Die Übergangszone wird also nun derart erzeugt, dass innerhalb einer begrenzten Anzahl von Umdrehungen der winkelabhängige Bahnabstand so moduliert wird, dass der Rand der Korrekturzone 34 hinsichtlich der z-Koordinate an die Flapfläche 19 herangeführt wird.

Die Verwendung gepulster Laserstrahlung ist nicht die einzige Art und Weise, wie die operative Refraktionskorrektur ausgeführt werden kann. Die hier beschriebene Bestimmung von Steuerdaten für den Betrieb der Vorrichtung kann vielmehr für nahezu jedes Operationsverfahren verwendet werden, bei dem mittels einer Vorrichtung unter Steuerung durch Steuerdaten ein Volumen aus der Augenhornhaut entfernt oder dieser, wie im allgemeinen Teil der Beschreibung bereits erläutert, hinzugefügt wird.

**Patentansprüche**

1. Vorrichtung zur Erzeugung von Steuerdaten zur Ansteuerung einer Lasereinrichtung (L) zur operativen Fehlsichtigkeitskorrektur eines Auges (3) eines Patienten (4), wobei

   - die Steuerdaten zur Ansteuerung einer Lasereinrichtung (L) ausgebildet sind, welche durch Einstrahlen von Laserstrahlung (2) in die Hornhaut (5) des Auges (3) Hornhaut-Gewebe trennt, und
   - die Vorrichtung (12) die Steuerdaten so erstellt, dass die Lasereinrichtung (L) bei Betrieb gemäß den Steuerdaten die Laserstrahlung (2) so abgibt, dass ein Volumen (18) innerhalb der Hornhaut (5) isoliert ist, dessen Entfernung aus der Hornhaut (5) die gewünschte Fehlsichtigkeitskorrektur bewirkt,
   - wobei die Vorrichtung (12) zur Ermittlung der Steuerdaten

      -- entweder einen Krümmungsradius Rcv* berechnet, den die um das Volumen (18) verminderte Hornhaut (5) hat, der ortsabhängig ist und der folgender Gleichung genügt: $R_{CV}^*(r,\varphi) = 1/((1/R_{CV}(r,\varphi)) + B_{COR}(r,\varphi)/(n_c-1)) + F$, wobei $R_{CV}(r,\varphi)$ der lokale Krümmungsradius der Hornhaut (5) vor Entfernung des Volumens (18),

$n_c$ die Brechzahl des Materials der Hornhaut (5), F ein Faktor ist, und $B_{COR}(r,\varphi)$ die für die gewünschte Fehlsichtigkeitskorrektur nötige, lokale Brechkraftänderung in einer im Scheitelpunkt der Hornhaut (5) liegenden Ebene ist,

-- oder eine Brechkraft $B^*_{CV}$ berechnet, die die um das Volumen (18) verminderte Hornhaut (5) hat, die ortsabhängig ist und die folgender Gleichung genügt:

$$B^*_{CV}(r,\varphi) = \cfrac{1}{\cfrac{1}{B_{CV}(r,\varphi) + B_{COR}(r,\varphi)} + \cfrac{F}{(n_C - 1)}}$$

wobei $B_{CV}(r,(p)$ die lokale Brechkraft der Hornhaut (5) vor Entfernung des Volumens (18) ist, und

- es in Polarkoordinaten gesehen für alle Winkel $\varphi$ mindestens zwei Radien r1 und r2 gibt, für die $B_{COR}(r=r1,\varphi) \neq B_{COR}(r=r2,\varphi)$ gilt,

**dadurch gekennzeichnet, dass**
die Vorrichtung bei der Ermittlung der Steuerdaten die lokale Brechkraftänderung $B_{COR}(r,\varphi)$ so festlegt, dass ein charakteristischer Radius $r_{ch}$ besteht, für den $B_{COR}(r<r_{ch},\varphi=const) = B_a \neq B_b = B_{COR}(r>r_{ch},\varphi=const)$ gilt.

2. Vorrichtung nach Anspruch 1, **dadurch gekennzeichnet, dass** die Vorrichtung (12) bei der Ermittlung der Steuerdaten für den Faktor $F = (1 - 1/n_c) \cdot \Delta z(r=0,\varphi)$ verwendet, wobei $\Delta z(r=0,\varphi)$ die zentrale Dicke des in der Hornhaut (5) zu isolierenden und entfernenden Volumens (18) ist und die Vorrichtung den ortsabhängigen Krümmungsradius $R_{CV}^*(r,\varphi)$ iterativ berechnet, indem sie bei jedem Iterationsschritt aus der Differenz der zentralen reziproken Krümmungsradien $1/R_{CV}^*(r=0,\varphi)$ und $1/R_{CV}(r=0,\varphi)$ auf die zentrale Dicke $\Delta z(r=0,\varphi)$ des Volumens (18) schließt und diesen Wert bei der Berechnung von $R_{CV}^*(r,\varphi)$ im nächsten Iterationsschritt anwendet.

3. Vorrichtung nach einem der Ansprüche 1 bis 2, **dadurch gekennzeichnet, dass** sie bei der Ermittlung der Steuerdaten die lokale Brechkraftänderung $B_{COR}(r,\varphi)$ so festlegt, dass ein zwei Radien $r_a$ und $r_b$ bestehen, für die $B_{COR}(r<r_a,\varphi=const) = B_a \neq B_b = B_{COR}(r>r_b,\varphi=const)$ gilt, wobei die Radialfunktion der Brechkraftänderung $B_{COR}(r,\varphi)$ im Übergangsbereich zwischen $r_a$ und $r_b$ kontinuierlich von $B_a$ nach $B_b$ übergeht.

4. Vorrichtung nach einem der Ansprüche 1 bis 3, wobei eine Korrekturfläche vorgegeben ist, die in der Hornhaut (5) zur Entfernung eines Volumens (18) als Schnittfläche (19, 20) erzeugt werden soll und die bezogen auf eine Haupteinfallsrichtung (A) der Laserstrahlung (2) nicht-rotationssymmetrisch ist, und

   - die Vorrichtung die Steuerdaten auf Basis der Korrekturfläche so erzeugt, dass die Laserbearbeitungsvorrichtung (L) im Betrieb die Korrekturfläche in der Hornhaut (5) erzeugt, und
   - die Vorrichtung bei der Erzeugung der Steuerdaten die nicht-rotationssymmetrische Korrekturfläche auf einen - in der Haupteinfallsrichtung (A) der Laserstrahlung (2) gesehen - kreisförmigen Umriss anpasst,
   - wobei die Vorrichtung bei der Erzeugung der Steuerdaten für die Korrekturfläche einen Übergangsbereich (35) vorsieht, in dem die Korrekturfläche von der nicht-rotationssymmetrischen Form auf einen, bezogen auf die Haupteinfallsrichtung (A) rotationssymmetrischen Rand angepasst wird, wobei optional der rotationssymmetrische Rand kreisförmig ist und in einer Ebene liegt, die senkrecht zur Haupteinfallsrichtung (A) steht und die bezogen auf die Haupteinfallsrichtung weder anteriorer als der anteriorste Punkt noch posteriorer als der posteriorste Punkt der Korrekturfläche ist.

5. Vorrichtung nach Anspruch 4, **dadurch gekennzeichnet, dass** die Laserbearbeitungsvorrichtung (L) zur Fokussierung der Laserstrahlung (2) in die Hornhaut (5) des Auges (3) längs der Haupteinfallsrichtung (A) und zur Verstellung der Lage des Laserfokus (6) in der Hornhaut (5) ausgebildet ist, und

   - die Vorrichtung bei der Erzeugung der Steuerdaten den rotationssymmetrischen Rand festlegt,
   - die Vorrichtung Steuerdaten erzeugt, die eine Bahn (32) vorgeben, entlang welcher der Laserfokus (6) zu verstellen ist, wobei die Bahn (32) in der vorgegebenen Korrekturfläche liegt und-spiralförmig von einem Inneren der vorgegeben Korrekturfläche zu deren Rand läuft, wobei
   - die Steuerdaten im Übergangsbereich (35) die Spirale so fortsetzen, dass diese pro Umlauf den Abstand zwischen dem Rand der vorgegeben Korrekturfläche und dem rotationssymmetrischen Rand gemäß einer vorbestimmten Funktion, vorzugsweise linear, verringert.

**6.** Verfahren zur Erzeugung von Steuerdaten zur Ansteuerung einer Lasereinrichtung (L) zur operativen Fehlsichtigkeitskorrektur eines Auges (3) eines Patienten (4), wobei

- die Steuerdaten zur Ansteuerung einer Lasereinrichtung (L) ausgebildet sind, welche durch Einstrahlen von Laserstrahlung (2) in die Hornhaut (5) des Auges (3) Hornhaut-Gewebe trennt, und
- die Steuerdaten so erstellt werden, dass die Lasereinrichtung (L) bei Betrieb gemäß den Steuerdaten die Laserstrahlung (2) so abgibt, dass ein Volumen (18) innerhalb der Hornhaut (5) isoliert ist, dessen Entfernung aus der Hornhaut (5) die gewünschte Fehlsichtigkeitskorrektur bewirkt,
- wobei zur Ermittlung der Steuerdaten

-- entweder ein Krümmungsradius Rcv* berechnet wird, den die um das Volumen (18) verminderte Hornhaut (5) hat, der ortsabhängig ist und der folgender Gleichung genügt: $R_{CV}{}^*(r,\varphi) = 1/((1/R_{CV}(r,\varphi) + B_{COR}(r,\varphi)/(n_c-1)) + F$, wobei $R_{CV}(r,\varphi)$ der lokale Krümmungsradius der Hornhaut (5) vor Entfernung des Volumens (18), $n_c$ die Brechkraft des Materials der Hornhaut (5), F ein Faktor ist, und $B_{COR}(r,\varphi)$ die für die gewünschte Fehlsichtigkeitskorrektur nötige, lokale Brechkraftänderung in einer im Scheitelpunkt der Hornhaut (5) liegenden Ebene ist,
-- oder eine Brechkraft $B_{CV}{}^*$ berechnet wird, die die um das Volumen (18) verminderte Hornhaut (5) hat, die ortsabhängig ist und die folgender Gleichung genügt:

$$B^*{}_{CV}(r,\varphi) = \cfrac{1}{\cfrac{1}{B_{CV}(r,\varphi) + B_{COR}(r,\varphi)} + \cfrac{F}{(n_c - 1)}}$$

wobei $B_{CV}(r,(p)$ die lokale Brechkraft der Hornhaut (5) vor Entfernung des Volumens (18) ist, und
- es in Polarkoordinaten gesehen für alle Winkel $\varphi$ mindestens zwei Radien r1 und r2 gibt, für die $B_{COR}(r=r1,\varphi) \neq B_{COR}(r=r2,\varphi)$,

**dadurch gekennzeichnet, dass**
bei der Ermittlung der Steuerdaten die lokale Brechkraftänderung $B_{COR}(r,\varphi)$ so festgelegt wird, dass ein charakteristischer Radius $r_{ch}$ besteht, für den $B_{COR}(r<r_{ch},\varphi=const) = B_a \neq B_b = B_{COR}(r>r_{ch},\varphi=const)$ gilt.

**7.** Verfahren nach Anspruch 6, **dadurch gekennzeichnet, dass** bei der Ermittlung der Steuerdaten für den Faktor F = $(1 - 1/n_c) \cdot \Delta z(r=0,\varphi)$ verwendet wird, wobei $\Delta z(r=0,\varphi)$ die zentrale Dicke des in der Hornhaut (5) zu isolierenden und entfernenden Volumens (18) ist und die Vorrichtung den ortsabhängigen Krümmungsradius $R_{CV}{}^*(r,\varphi)$ iterativ berechnet, indem sie bei jedem Iterationsschritt aus der Differenz der zentralen reziproken Krümmungsradien $1/R_{CV}{}^*(r=0,\varphi)$ und $1/R_{CV}(r=0,(p)$ auf die zentrale Dicke $\Delta z(r=0,\varphi)$ des Volumens (18) schließt und diesen Wert bei der Berechnung von $R_{CV}{}^*(r,\varphi)$ im nächsten Iterationsschritt anwendet.

**8.** Verfahren nach einem der Ansprüche 6 oder 7, **dadurch gekennzeichnet, dass** bei der Ermittlung der Steuerdaten die lokale Brechkraftänderung $B_{COR}(r,\varphi)$ so festgelegt wird, dass ein zwei Radien $r_a$ und $r_b$ bestehen, für die $B_{COR}(r<r_a,\varphi=const) = B_a \neq B_b = B_{COR}(r>r_b,\varphi=const)$ gilt, wobei die Radialfunktion der Brechkraftänderung $B_{COR}(r,\varphi)$ im Übergangsbereich zwischen $r_a$ und $r_b$ kontinuierlich von $B_a$ nach $B_b$ übergeht.

**9.** Verfahren nach einem der Ansprüche 6 bis 8 **dadurch gekennzeichnet, dass** eine Korrekturfläche vorgegeben ist, die in der Hornhaut (5) zur Entfernung eines Volumens (18) erzeugt werden soll und die bezogen auf eine Haupteinfallsrichtung (A) der Laserstrahlung (2) nicht-rotationssymmetrisch ist, und wobei im Verfahren

- die Steuerdaten auf Basis der Korrekturfläche so erzeugt werden, dass die Laserbearbeitungsvorrichtung (L) im Betrieb die Korrekturfläche als Schnittfläche (19, 20) in der Hornhaut (5) erzeugt,
- die nicht-rotationssymmetrische Korrekturfläche auf einen - in der Haupteinfallsrichtung (A) der Laserstrahlung (2) gesehen - kreisförmigen Umriss angepasst wird, und
- für die Korrekturfläche ein Übergangsbereich (35) vorgesehen wird, in dem sie von der nicht-rotationssymmetrischen Form auf einen, bezogen auf die Haupteinfallsrichtung (A) rotationssymmetrischen Rand angepasst wird, wobei optional der rotationssymmetrische Rand kreisförmig ist und in einer Ebene liegt, die senkrecht zur Haupteinfallsrichtung steht und die bezogen auf die Haupteinfallsrichtung weder anteriorer als der anteriorste Punkt noch posteriorer als der posteriorste Punkt der Korrekturfläche ist.

10. Verfahren nach Anspruch 9, **dadurch gekennzeichnet, dass** die Laserbearbeitungsvorrichtung (L) zur Fokussierung von Bearbeitungs-Laserstrahlung in die Hornhaut (5) des Auges (3) längs der Haupteinfallsrichtung (A) und zur Verstellung der Lage des Laserfokus in der Hornhaut (5) ausgebildet ist, und

- der rotationssymmetrische Rand festgelegt wird,
- die Steuerdaten so erzeugt werden, dass sie eine Bahn (32) vorgeben, entlang welcher der Laserfokus (6) zu verstellen ist, wobei die Bahn (32) in der vorgegebenen Korrekturfläche liegt und spiralförmig von einem Inneren der vorgegeben Korrekturfläche zu deren Rand läuft, wobei
- die Steuerdaten im Übergangsbereich (35) die Spirale so fortsetzen, dass diese pro Umlauf den Abstand zwischen dem Rand der vorgegeben Korrekturfläche und dem rotationssymmetrischen Rand gemäß einer vorbestimmten Funktion, vorzugsweise linear, verringert.

11. Vorrichtung nach Anspruch 4 oder Verfahren nach Anspruch 9, wobei der Übergangsbereich (35) eine ebene Fläche ist, die senkrecht zur Haupteinfallsrichtung (A) liegt und die an die Korrekturfläche anschließt und diese auf den kreisförmigen Umriss ergänzt.

12. Vorrichtung oder Verfahren nach Anspruch 11, wobei die Steuerdaten eine Bahn (32) vorgeben, entlang welcher der Laserfokus (6) zu verstellen ist, wobei die Bahn (32) in der vorgegebenen Korrekturfläche liegt und spiralförmig von einem Inneren der vorgegeben Korrekturfläche zu deren Rand läuft, und die Steuerdaten im Übergangsbereich (35) die Bahn (32) als in der ebenen Fläche liegende Spirale oder konzentrische Kreise festlegen, und die für diejenigen Abschnitte der Spirale oder konzentrischen Kreise, die in Sichtweise längs zur Haupteinfallsrichtung (A) mit der Korrekturfläche überlappen würden, ein Deaktivieren der Laserstrahlung (2) hinsichtlich deren Bearbeitungswirkung vorsehen.

13. Computerprogrammprodukt mit Programmcode, der bei Ausführung auf einem Computer (P) ein Verfahren gemäß einem der Ansprüche 6 bis 12 durchführt.

**Claims**

1. A device for generating control data for controlling a laser (L) for surgical correction of defective vision of an eye (3) of a patient (4), wherein

- the control data are adapted to control a laser (L) which cuts corneal tissue by irradiating laser radiation (2) into the cornea (5) of the eye (3), and
- the device (12) generates the control data such that the laser (L), during operation according to the control data, emits the laser radiation (2) such that a volume (18) in the cornea (5) is isolated, the removal of which volume from the cornea (5) effects the desired correction of defective vision,
- wherein to determine the control data, the device (12)

-- either calculates a radius of curvature $R_{CV}^*$ which radius the cornea (5) has without the volume (18) and which radius is locally varying and satisfies the following equation: $R_CV^*(r,\varphi) = 1 / ( (1/R_{CV}(r,\varphi)) + B_{COR}(r,\varphi)/(n_c-1)) + F$, wherein $R_{CV}(r,\varphi)$ is the local radius of curvature of the cornea (5) before the volume (18) is removed, $n_c$ is the refractive index of the material of the cornea (5), F is a coefficient, and $B_{COR}(r,\varphi)$ is the local change in optical refraction power in a plane lying in the vertex of the cornea (5) and required for the desired correction of defective vision,
-- or calculates an optical refraction power $B^*_{CV}$ which optical refraction power the cornea (5) has without the volume (18) and which radius is locally varying and satisfies the following equation:

$$B^*_{CV}(r,\varphi) = \cfrac{1}{\cfrac{1}{B_{CV}(r,\varphi) + B_{COR}(r,\varphi)} + \cfrac{F}{(n_C - 1)}}$$

wherein $B_{CV}(r,\varphi)$ is the local optical refraction power of the cornea (5) before the volume (18) is removed, and

- seen in polar coordinates, there are at least two radii, r1 and r2 for all angles $\varphi$, for which $B_{COR}(r=r1,\varphi) \neq B_{COR}(r=r2,\varphi)$ holds true,

**characterized in that**
when determining the control data, the device defines the local change in optical refraction power $B_{COR}(r,\varphi)$ such that there is a characteristic radius $r_{ch}$ for which radius thus $B_{COR}(r<r_{ch},\varphi=const) = B_a \neq B_b = B_{COR}(r>r_{ch},\varphi=const)$ holds true.

2. The device according to claim 1, **characterized in that** the device (12) uses $F = (1 - 1/n_c) \cdot \Delta z(r=0,\varphi)$ for the coefficient F, wherein $\Delta z(r=0,\varphi)$ is the central thickness of the volume (18) to be isolated in the cornea (5) and removed and the device iteratively calculates the locally varying radius of curvature $R_{CV}^*(r,\varphi)$, wherein the device derives in each iteration step the central thickness $\Delta z(r=0,\varphi)$ of the volume (18) from the difference between the central reciprocal radii of curvature $1/R_{CV}^*(r=0,\varphi)$ and $1/R_{CV}(r=0,\varphi)$, and applies this value when calculating $R_{CV}^*(r,\varphi)$ in the next iteration step.

3. The device according to any of claims 1 to 2, **characterized in that**, when determining the control data, the device defines the local change in optical refraction power $B_{COR}(r,\varphi)$ such that there are two radii $r_a$ and $r_b$, for which radii thus $B_{COR}(r<r_a,\varphi=const) = B_a \neq B_b = B_{COR}(r>r_b,\varphi=const)$ holds true, wherein the radial function of the change in optical refraction power $B_{COR}(r,\varphi)$ changes continuously from $B_a$ to $B_b$ in a transition region between $r_a$ and $r_b$.

4. The device according to any of claims 1 to 3, wherein a correction surface is predetermined which is to be produced as cut surface (19, 20) in the cornea (5) for the removal of a volume (18) and which is non-rotation-symmetrical relative to a main direction of incidence (A) of the laser radiation (2), and

- the device generates the control data on the basis of the correction surface such that, during operation, the laser treatment device (L) produces the correction surface in the cornea (5), and
- when generating the control data the device adapts the non-rotation-symmetrical correction surface to a contour that is circular when viewed in the main direction of incidence (A) of the laser radiation (2),
- wherein when generating the control data, the device provides a transition region (35) for the correction surface, in which transition region the correction surface is adapted from the non-rotation-symmetrical form to an edge being rotation-symmetrical relative to the main direction of incidence (A), wherein optionally, the rotation-symmetrical edge is circular and lies in a plane which is perpendicular to the main direction of incidence (A) and which, relative to the main direction of incidence, is neither more anterior than a most anterior point nor more posterior than a most posterior point of the correction surface.

5. The device according to claim 4, **characterized in that** the laser treatment device (L) is adapted to focus the laser radiation (2) into the cornea (5) of the eye (3) along the main direction of incidence (A) and for shifting the position of the laser focus (6) in the cornea (5), and

- when generating the control data, the device defines the rotation-symmetrical edge,
- the device generates the control data which define a path (32) along which the laser focus (6) is to be shifted, wherein the path (32) lies in the predetermined correction surface and runs spirally from a center of the predetermined correction surface to an edge of the predetermined correction surface, wherein
- the control data in the transition region (35) continue the spiral path such that in each revolution of the spiral path the distance between the edge of the predetermined correction surface and the rotation-symmetrical edge is reduced according to a predetermined function, preferably linearly.

6. A method for generating control data for controlling a laser (L) for surgical correction of defective vision of an eye (3) of a patient (4), wherein

- the control data are adapted to control a laser (L) which cuts corneal tissue by irradiating laser radiation (2) into the cornea (5) of the eye (3),
- the control data are generated such that the laser (L), during operation according to the control data, emits the laser radiation (2) such that a volume (18) in the cornea (5) is isolated, the removal of which volume from the cornea (5) effects the desired correction of defective vision,
- wherein to determine the control data,

-- either a radius of curvature Rcv$^*$ is calculated which radius the cornea (5) has without the volume (18) and which radius is locally varying and satisfies the following equation: $R_CV^*(r,\varphi) = 1 / ((1/R_{CV}(r,\varphi)) + B_{COR}(r,\varphi)/(n_c-1)) + F$, wherein $R_{CV}(r,\varphi)$ is the local radius of curvature of the cornea (5) before the volume (18) is removed, $n_c$ is the refractive index of the material of the cornea (5), F is a coefficient, and $B_{COR}(r,\varphi)$ is the local change in optical refraction power in a plane lying in the vertex of the cornea (5) and required for the desired correction of defective vision,

-- or an optical refraction power B$^*_{CV}$ is calculated which optical refraction power the cornea (5) has without the volume (18) and which radius is locally varying and satisfies the following equation:

$$B^*_{CV}(r,\varphi) = \cfrac{1}{\cfrac{1}{B_{CV}(r,\varphi) + B_{COR}(r,\varphi)} + \cfrac{F}{(n_c - 1)}}$$

wherein $B_{CV}(r,\varphi)$ is the local optical refraction power of the cornea (5) before the volume (18) is removed, and

- seen in polar coordinates, there are at least two radii, r1 and r2 for all angles $\varphi$, for which $B_{COR}(r=r1,\varphi) \neq B_{COR}(r=r2,\varphi)$ holds true,

**characterized in that**
when determining the control data, the local change in optical refraction power $B_{COR}(r,\varphi)$ is defined such that there is a characteristic radius $r_{ch}$ for which radius thus $B_{COR}(r<r_{ch},\varphi=const) = B_a \neq B_b = B_{COR}(r>r_{ch},\varphi=const)$ holds true.

7. The method according to claim 6, **characterized in that** $F = (1 - 1/nc) \cdot \Delta z(r=0,\varphi)$ is used for the coefficient F when determining the control data, wherein $\Delta z(r=0,\varphi)$ is the central thickness of the volume (18) to be isolated in the cornea (5) and removed and the locally varying radius of curvature $R_{CV}^*(r,\varphi)$ is iteratively calculated, wherein in each iteration step the central thickness $\Delta z(r=0,\varphi)$ of the volume (18) is determined from the difference between the central reciprocal radii of curvature $1/R_{CV}^*(r=0,\varphi)$ and $1/R_{CV}(r=0,\varphi)$, and this value is applied when calculating $R_{CV}^*(r,\varphi)$ in a next iteration step.

8. The method according to any of claims 6 or 7, **characterized in that**, when determining the control data, the local change in optical refraction power $B_{COR}(r,\varphi)$ is defined such that there are two radii $r_a$ and $r_b$, for which thus $B_{COR}(r<r_a,\varphi=const) = B_a \neq B_b = B_{COR}(r>r_b,\varphi=const)$ holds true, wherein the radial function of the change in optical refraction power $B_{COR}(r,\varphi)$ changes continuously from $B_a$ to $B_b$ in a transition region between $r_a$ and $r_b$.

9. A method according to any of claims 6 to 8, **characterized in that** a correction surface is predetermined which is to be produced in the cornea (5) for the removal of a volume (18) and which is non-rotation-symmetrical relative to the main direction of incidence (A) of the laser radiation (2), and wherein in the method

- the control data are generated on the basis of the correction surface such that, during operation, the laser treatment device (L) produces the correction surface as a cut surface (19, 20) in the cornea (5), and
- the non-rotation-symmetrical correction surface is adapted to a contour that is circular when viewed in a main direction of incidence (A) of the laser radiation (2), and
- a transition region (35) is provided for the correction surface in which transition region the correction surface is adapted from the non-rotation-symmetrical form to an edge being rotation-symmetrical relative to the main direction of incidence (A), wherein optionally, the rotation-symmetrical edge is circular and lies in a plane which is perpendicular to the main direction of incidence and which, relative to the main direction of incidence, is neither more anterior than a most anterior point nor more posterior than a most posterior point of the correction surface.

10. The method according to claim 9, **characterized in that** the laser treatment device (L) is adapted for focusing the laser radiation into the cornea (5) of the eye (3) along the main direction of incidence (A) and for shifting the position of the laser focus in the cornea (5), and

- the rotation-symmetrical edge is defined,
- the control data are generated such that they define a path (32) along which the laser focus (6) is to be shifted, wherein the path (32) lies in the predetermined correction surface and runs spirally from a center of the predetermined correction surface to an edge of the predetermined correction surface, wherein
- the control data continue the spiral path in the transition region (35) such that in each revolution of the spiral path the distance between the edge of the predetermined correction surface and the rotation-symmetrical edge is reduced according to a predetermined function, preferably linearly.

11. The device according to claim 4 or method according to claim 9, **characterized in that** the transition region is a flat surface which lies perpendicular to the main direction of incidence (A) and which joins onto the correction surface and completes this to the circular contour.

12. The device or method according to claim 11, the control data define a path (32) along which the laser focus (6) is to be shifted, wherein the path (32) lies in the predetermined correction surface and runs spirally from a center of the predetermined correction surface to an edge of the predetermined correction surface, and the control data define the path (32) in the transition region (35) as a spiral lying in the flat surface or as concentric circles, and wherein the control data provide a deactivation of the laser radiation (2) with regard to its processing effect for those sections of the spirals or concentric circles lying in the flat surface which would overlap with the correction surface when looking along the main direction of incidence (A).

13. A computer program product with program code which carries out a method according to any of claims 6 to 12 when being executed on a computer (P).

**Revendications**

1. Dispositif pour générer des données de commande destinées à commander un appareil à laser (L) servant à la correction chirurgicale d'une déficience visuelle d'un œil (3) d'un patient (4),

- les données de commande étant conçues pour exciter un appareil à laser (L), lequel sépare du tissu cornéen par irradiation avec le rayon laser (2) dans la cornée (5) de l'œil (3), et
- le dispositif (12) créant les données de commande de telle sorte que l'appareil à laser (L), lors du fonctionnement, délivre le rayonnement laser (2) conformément aux données de commande de telle sorte qu'à l'intérieur de la cornée (5) un volume (18) est isolé dont l'enlèvement de la cornée (5) produit la correction souhaitée de la déficience visuelle,
- dans lequel l'arrangement (12), en vue de déterminer les données de commande, calcule

-- soit un rayon de courbure $R_{CV}^*$ que présente la cornée (5) réduite du volume (18), lequel est dépendant du lieu et obéit à l'équation suivante : $R_{CV}^*(r,\varphi) = 1/((1/R_{CV}(r,\varphi)) + B_{COR}(r,\varphi)/(n_c-1)) + F$, $R_{CV}(r,\varphi)$ désignant le rayon de courbure local de la cornée (5) avant l'enlèvement du volume (18), $n_c$ la puissance de réfraction de la matière de la cornée (5), F un facteur et $B_{COR}(r,\varphi)$ la modification locale de la puissance de réfraction nécessaire pour la correction souhaitée de la déficience visuelle dans un plan qui se trouve dans le sommet de la cornée (5),
-- soit une puissance de réfraction $B_{CV}^*$ que présente la cornée (5) réduite du volume (18), laquelle est dépendante du lieu et obéit à l'équation suivante :

$$B_{CV}^*(r,\varphi) = \frac{1}{\frac{1}{B_{CV}(r,\varphi)+B_{COR}(r,\varphi)} + \frac{F}{(n_c-1)}}$$

$B_{CV}(r,\varphi)$ désignant la puissance de réfraction locale de la cornée (5) avant l'enlèvement du volume (18), et

- pour tous les angles $\varphi$, vus dans les coordonnées polaires, au moins deux rayons r1 et r2 étant définis pour lesquels $B_{COR}(r=r1,\varphi) \neq B_{COR}(r=r2,\varphi)$,

**caractérisé en ce que**
lors de la détermination des données de commande, le dispositif fixe la modification locale de la puissance de réfraction $B_{COR}(r,\varphi)$ de telle sorte qu'il est obtenu un rayon caractéristique $r_{ch}$ pour lequel s'applique $B_{COR}(r<r_{ch},\varphi=const) = B_a \neq B_b = B_{COR}(r>r_{ch},\varphi=const)$.

2. Dispositif selon la revendication 1, **caractérisé en ce que** le dispositif (12), lors de la détermination des données de commande, utilise pour le facteur F = $(1 - 1/n_c)$ * $\Delta z(r=0,\varphi)$, $\Delta z(r=0,\varphi)$ désignant l'épaisseur centrale du volume (18) à isoler et à retirer dans la cornée (5) et le dispositif calculant le rayon de courbure $R_{CV}$*$(r,\varphi)$ dépendant du lieu de manière itérative **en ce qu'**à chaque étape d'itération, il déduit l'épaisseur centrale $\Delta z(r=0,\varphi)$ du volume (18) à partir de la différence entre les rayons de courbure réciproques centraux $1/R_{CV}$*$(r=0,\varphi)$ et $1/R_{CV}(r=0,\varphi)$ et applique cette valeur lors du calcul de $R_{CV}$*$(r,\varphi)$ dans l'étape d'itération suivante.

**3.** Dispositif selon l'une des revendications 1 et 2, **caractérisé en ce que** lors de la détermination des données de commande, il définit la modification locale de la puissance de réfraction $B_{COR}(r,\varphi)$ de telle sorte que sont obtenus deux rayons $r_a$ et $r_b$, pour lesquels s'applique $B_{COR}(r<r_a,\varphi=const) = B_a \neq B_b = B_{COR}(r>rb,\varphi=const)$, la fonction radiale de la modification de la puissance de réfraction $B_{COR}(r,\varphi)$ dans la zone de transition entre $r_a$ et $r_b$ passant de manière continue de $B_a$ à $B_b$.

**4.** Dispositif selon l'une des revendications 1 à 3, une surface de correction étant prédéfinie, laquelle doit être générée dans la cornée (5) en tant que surface de coupe (19, 20) en vue d'enlever un volume (18) et laquelle n'est pas symétrique en rotation en référence à une direction d'incidence principale (A) du rayon laser (2), et

- le dispositif générant les données de commande en se basant sur la surface de correction de telle sorte que l'appareil de traitement au laser (L), en fonctionnement, génère la surface de correction dans la cornée (5), et
- le dispositif, lors de la génération des données de commande, adaptant la surface de correction non symétrique en rotation à un contour qui, vu dans la direction d'incidence principale (A) du rayon laser (2), est circulaire,
- le dispositif, lors de la génération des données de commande, prévoyant pour la surface de correction une zone de transition (35) dans laquelle la surface de correction est adaptée de la forme non symétrique en rotation à un bord symétrique en rotation en référence à la direction d'incidence principale (A), le bord symétrique en rotation étant facultativement circulaire et se trouvant dans un plan qui est perpendiculaire à la direction d'incidence principale (A) et qui, en référence à la direction d'incidence principale, n'est ni plus antérieur au point le plus antérieur, ni plus postérieur au point le plus postérieur de la surface de correction.

**5.** Dispositif selon la revendication 4, **caractérisé en ce que** l'appareil de traitement au laser (L) est configuré pour concentrer le rayon laser (2) dans la cornée (5) de l'œil (3) le long de la direction d'incidence principale (A) et pour déplacer la position du point focal du laser (6) dans la cornée (5), et

- le dispositif, lors de la génération des données de commande, définit le bord symétrique en rotation,
- le dispositif génère des données de commande qui prédéfinissent une trajectoire (32) le long de laquelle il faut déplacer le point focal du laser (6), la trajectoire (32) se trouvant dans la surface de correction prédéfinie et s'étendant sous la forme d'une spirale depuis un intérieur de la surface de correction prédéfinie jusqu'à son bord,
- les données de commande poursuivant la spirale dans la zone de transition (35) de telle sorte que celle-ci réduit à chaque tour l'écart entre le bord de la surface de correction prédéfinie et le bord à symétrie de rotation conformément à une fonction prédéterminée, de préférence linéaire.

**6.** Procédé pour générer des données de commande destinées à commander un appareil à laser (L) servant à la correction chirurgicale d'une déficience visuelle d'un œil (3) d'un patient (4),

- les données de commande étant conçues pour exciter un appareil à laser (L), lequel sépare du tissu cornéen par irradiation avec le rayon laser (2) dans la cornée (5) de l'œil (3), et
- les données de commande étant créées de telle sorte que l'appareil à laser (L), lors du fonctionnement, délivre le rayonnement laser (2) conformément aux données de commande de telle sorte qu'à l'intérieur de la cornée (5) un volume (18) est isolé dont l'enlèvement de la cornée (5) produit la correction souhaitée de la déficience visuelle,
- en vue de déterminer les données de commande, il est calculé

-- soit un rayon de courbure $R_{CV}$* que présente la cornée (5) réduite du volume (18), lequel est dépendant du lieu et obéit à l'équation suivante : $R_{CV}$*$(r,\varphi) = 1/((1/R_{CV}(r,\varphi)) + B_{COR}(r,\varphi)/(n_c-1)) + F$, $R_{CV}(r,\varphi)$ désignant le rayon de courbure local de la cornée (5) avant l'enlèvement du volume (18), $n_c$ la puissance de réfraction de la matière de la cornée (5), F un facteur et $B_{COR}(r,\varphi)$ la modification locale de la puissance de réfraction nécessaire pour la correction souhaitée de la déficience visuelle dans un plan qui se trouve dans le sommet de la cornée (5),
-- soit une puissance de réfraction $B_{CV}$* que présente la cornée (5) réduite du volume (18), laquelle est dépendante du lieu et obéit à l'équation suivante :

$$B^*_{CV}(r,\varphi) = \cfrac{1}{\cfrac{1}{B_{CV}(r,\varphi)+B_{COR}(r,\varphi)}+\cfrac{F}{(n_C-1)}}$$

$B_{CV}(r,\varphi)$ désignant la puissance de réfraction locale de la cornée (5) avant l'enlèvement du volume (18), et

- pour tous les angles $\varphi$, vus dans les coordonnées polaires, au moins deux rayons r1 et r2 étant définis pour lesquels $B_{COR}(r=r1,\varphi) \neq B_{COR}(r=r2,\varphi)$,

**caractérisé en ce que**
lors de la détermination des données de commande, la modification locale de la puissance de réfraction $B_{COR}(r,\varphi)$ est fixée de telle sorte qu'il est obtenu un rayon caractéristique $r_{ch}$ pour lequel s'applique $B_{COR}(r<r_{ch},\varphi=const) = B_a \neq B_b = B_{COR}(r>r_{ch},\varphi=const)$.

7. Procédé selon la revendication 6, **caractérisé en ce que** lors de la détermination des données de commande, F = $(1 - 1/n_c) \cdot \Delta z(r=0,\varphi)$ est utilisé pour le facteur F, $\Delta z(r=0,\varphi)$ désignant l'épaisseur centrale du volume (18) à isoler et à retirer dans la cornée (5) et le dispositif calculant le rayon de courbure $R_{CV}^*(r,\varphi)$ dépendant du lieu de manière itérative **en ce qu'**à chaque étape d'itération, il déduit l'épaisseur centrale $\Delta z(r=0,\varphi)$ du volume (18) à partir de la différence entre les rayons de courbure réciproques centraux $1/R_{CV}^*(r=0,\varphi)$ et $1/R_{CV}(r=0,\varphi)$ et applique cette valeur lors du calcul de $R_{CV}^*(r,\varphi)$ dans l'étape d'itération suivante.

8. Procédé selon l'une des revendications 6 et 7, **caractérisé en ce que** lors de la détermination des données de commande, la modification locale de la puissance de réfraction $B_{COR}(r,\varphi)$ est définie de telle sorte que sont obtenus deux rayons $r_a$ et $r_b$, pour lesquels s'applique $B_{COR}(r<r_a,\varphi=const) = B_a \neq B_b = B_{COR}(r>r_b,\varphi=const)$, la fonction radiale de la modification de la puissance de réfraction $B_{COR}(r,\varphi)$ dans la zone de transition entre $r_a$ et $r_b$ passant de manière continue de $B_a$ à $B_b$.

9. Procédé selon l'une des revendications 6 à 8, **caractérisé en ce qu'**une surface de correction est prédéfinie, laquelle doit être générée dans la cornée (5) en vue d'enlever un volume (18) et laquelle n'est pas symétrique en rotation en référence à une direction d'incidence principale (A) du rayon laser (2) et, dans le procédé,

- les données de commande étant générées en se basant sur la surface de correction de telle sorte que l'appareil de traitement au laser (L), en fonctionnement, génère la surface de correction en tant que surface de coupe (19, 20) dans la cornée (5), et
- la surface de correction non symétrique en rotation étant adaptée à un contour qui, vu dans la direction d'incidence principale (A) du rayon laser (2), est circulaire, et
- une zone de transition (35) étant prévue pour la surface de correction, dans laquelle elle est adaptée de la forme non symétrique en rotation à un bord symétrique en rotation en référence à la direction d'incidence principale (A), le bord symétrique en rotation étant facultativement circulaire et se trouvant dans un plan qui est perpendiculaire à la direction d'incidence principale et qui, en référence à la direction d'incidence principale, n'est ni plus antérieur au point le plus antérieur, ni plus postérieur au point le plus postérieur de la surface de correction.

10. Procédé selon la revendication 9, **caractérisé en ce que** l'appareil de traitement au laser (L) est configuré pour concentrer le rayon laser de traitement dans la cornée (5) de l'œil (3) le long de la direction d'incidence principale (A) et pour déplacer la position du point focal du laser dans la cornée (5), et

- le bord symétrique en rotation est défini,
- les données de commande sont générées de telle sorte qu'elles prédéfinissent une trajectoire (32) le long de laquelle il faut déplacer le point focal du laser (6), la trajectoire (32) se trouvant dans la surface de correction prédéfinie et s'étendant sous la forme d'une spirale depuis un intérieur de la surface de correction prédéfinie jusqu'à son bord,
- les données de commande poursuivant la spirale dans la zone de transition (35) de telle sorte que celle-ci réduit à chaque tour l'écart entre le bord de la surface de correction prédéfinie et le bord à symétrie de rotation conformément à une fonction prédéterminée, de préférence linéaire.

**11.** Dispositif selon la revendication 4 ou procédé selon la revendication 9, la zone de transition (35) étant une surface plane qui est perpendiculaire à la direction d'incidence principale (A) et qui est rattachée à la surface de correction et complète celle-ci sur le contour circulaire.

**12.** Dispositif ou procédé selon la revendication 11, les données de commande prédéfinissant une trajectoire (32) le long de laquelle il faut déplacer le point focal du laser (6), la trajectoire (32) se trouvant dans la surface de correction prédéfinie et s'étendant sous la forme d'une spirale depuis un intérieur de la surface de correction prédéfinie jusqu'à son bord, et les données de commande définissant la trajectoire (32) dans la zone de transition (35) sous la forme d'une spirale ou de cercles concentriques se trouvant dans la surface plane, et prévoyant pour les portions de la spirale ou les cercles concentriques respectifs, qui chevaucheraient la surface de correction visuellement le long de la direction d'incidence principale (A), une désactivation du rayon laser (2) du point de vue de son effet de traitement.

**13.** Programme informatique comprenant un code de programme qui, lors de l'exécution sur un ordinateur (P), met en œuvre un procédé selon l'une des revendications 6 à 12.

FIG 1

FIG 3

Fig. 5

Fig. 6

Fig. 2

FIG 4

12

8

~2

9

10

11

5

7

5 ~

18

$d_F$

15

$d_L$

$h_F$

$h_L$

19

20

Fig. 7

FIG 8

Fig. 9

Fig. 10

S1 — Topographie der Kornea $Z_{c_0}(r,\varphi)$

S2 — Lokale Krümmung $R_{cv}(r,\varphi)$

S3 — Lokale Brechkraft $B_{cv}(r,\varphi)$

S4 — Lokale Brechkraft nach Korrektur $B^*_{cv}(r,\varphi) = B_{cv}(r,\varphi) + B_{cor}(r,\varphi)$

S5 — Neuer lokaler Krümmungsradius $R^*_{cv}(r,\varphi)$

S6 — Volumenbegrenzung muss Krümmungsänderung umsetzen

S7 — Veränderung der Topographie der Kornea $Z_{cv}^*(r,\varphi) = Z_{cv}(r,\varphi) + \Delta Z(r,\varphi)$

— Diagnose Daten

— Diagnose Daten (optional)

— Daten zur refraktiven Korrektur

Vorgabe der Grundform des Volumens

Fig. 11

Fig 12

Fig. 14

Fig. 13

Fig. 15

Fig. 16

Fig. 17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9611655 A
- EP 1153584 A1
- US 5993438 A
- DE 102005013558 A1
- WO 2005092172 A
- US 6110166 A
- US 7131968 B2
- DE 102006053118 A1

- DE 102006053120 A1
- DE 102006053119 A1
- DE 10334110 A1
- EP 1159986 A1
- US 5549632 A
- DE 69500997 T2
- WO 2004032810 A2
- DE 10358927 A1